# EUROPEAN PATENT APPLICATION

(11) **EP 4 763 118 A1**
(43) Date of publication of application: **24.06.2026**
(21) Application number: 25222238.5
(22) Date of filing: 10.12.2025
(51) Int. Cl.: A61B 34/10, A61B 34/00, B33Y 80/00, A61B 17/00

(54) **SYSTEMS AND METHODS FOR GENERATING PATIENT-SPECIFIC IMPLANTS FOR ANATOMICAL STRUCTURES OF PATIENTS**

(30) Priority: 20.12.2024 US 202418990047
(71) Applicant: Immersivetouch, Inc., Chicago, Illinois 60607 (US)
(72) Inventor: LUO, Jia, CHICAGO, 60607 (US); DAMLE, Shireen, CHICAGO, 60607 (US); GHOSH, Tanvi, CHICAGO, 60607 (US); BANERJEE, Pat, CHICAGO, 60607 (US); BANERJEE, Jay, CHICAGO, 60607 (US)
(74) Representative: Harden, Henry Simon

(57) **Abstract**

Embodiments for generating patient-specific implants for anatomical structures of patients are disclosed. One embodiment includes a method that includes receiving image data of an anatomical structure of a patient, where the image data indicates a fracture site associated with the anatomical structure; receiving manipulation data associated with manipulation of one or more parameters associated with the fracture site in a virtual reality environment; visualizing a three-dimensional (3D) model of the anatomical structure based on the image data, the one or more parameters, and the manipulation data, where the 3D model is visualized in a 3D space; generating a set of instructions for a printer based on the 3D model, and controlling the printer for generating a 3D printed model of the anatomical structure based on the set of instructions. The 3D printed model is configured to receive an implant, such that the implant is contoured to match the fracture site.

## Description

### TECHNICAL FIELD

The present application generally relates to systems and methods for visualizing, planning, or performing a medical procedure, and more particularly to systems and methods for generating patient-specific implants for anatomical structures of patients.

### BACKGROUND

The use of immersive technologies such as augmented reality (AR), virtual reality (VR), and mixed reality (MR) is gradually becoming a standard in modern healthcare. These technologies enable the creation of a three-dimensional (3D) model of a patient anatomy from medical imaging data, allowing for customized surgical planning tailored to individual patients, particularly in orthopedic and reconstructive fields. The 3D model of the patient's anatomy typically provides measurements of an intended anatomical structure such as bones and tissues. The measurements assist in designing implants and other similar restorative devices for placement on the anatomical structure. While these 3D models represent the external shape and dimensions of the anatomical structure, they often fail to capture internal gaps, irregularities, or misalignments in connected tissues and bone structures, especially in cases of fractures or degenerative conditions. Consequently, the implants often require extensive customization or intraoperative adjustments to achieve proper positioning and fixation.

During surgical procedures, surgeons typically rely on manual adjustments to address mismatches or misalignments between the anatomical structure and an implant. The adjustments involve the use of additional fixtures, manual realignments, or modifications to the implant itself. This manual process is labor-intensive and generally requires multiple iterations to achieve the correct fit and alignment of the restorative device, leading to extended surgery times. Such prolonged surgical procedures increase the risk of complications, including excessive blood loss, infection, and fatigue for both the surgical team and the patient, in addition to a significant strain on healthcare resources, extended recovery times, and may result in suboptimal placement of the implant. Misaligned or improperly installed implant can suffer increased wear, mechanical instability, or failure, potentially necessitating revision surgeries, thus burdening the healthcare systems and negatively affecting patient recovery and related outcomes.

Therefore, there exists a need for an efficient and accurate approach to generating patient-specific implants for anatomical structures that reduce implementation time, minimize complications, and improve patient outcomes.

### SUMMARY

One embodiment of the present application includes a system including a memory, multiple controllers, an interaction interface, a graphics rendering module, and a three-dimensional (3D) module. The memory may be configured to store computer-executable instructions. The controllers may be configured to execute the instructions to: receive image data of an anatomical structure of a patient, where the image data indicates a fracture site associated with the anatomical structure; and identify one or more parameters associated with the fracture site based on the image data. The interaction interface may be configured to receive manipulation data from one or more augmented reality or virtual reality (AR/VR) devices, where the manipulation data is associated with manipulation of the one or more parameters associated with the fracture site in a virtual reality environment. The one or more parameters are manipulated to reduce an intensity of the fracture site. The graphics rendering module may be configured to: receive the image data, the one or more parameters, and the manipulation data, visualize a three-dimensional (3D) model of the anatomical structure based on the image data, the one or more parameters, and the manipulation data, where the 3D model of the patient is visualized in a 3D space using a plurality of graphics rendering mechanisms, and display the visualization of the 3D model on at least one of: a display device, or a GUI. The 3D printing module may be configured to: generate a set of instructions for a printer based on the 3D model, and control the printer for generating a 3D printed model of the anatomical structure based on the set of instructions, where the 3D printed model is configured to receive an implant, such that the implant is contoured to match the fracture site of the anatomical structure.

Another embodiment of the present application includes a method that includes receiving image data of an anatomical structure of a patient, where the image data indicates a fracture site associated with the anatomical structure; identifying one or more parameters associated with the fracture site based on the image data; receiving manipulation data from one or more augmented reality or virtual reality (AR/VR) devices, where the manipulation data is associated with manipulation of the one or more parameters associated with the fracture site in a virtual reality environment. The one or more parameters are manipulated to reduce an intensity of the fracture site. The method further includes visualizing a three-dimensional (3D) model of the anatomical structure based on the image data, the one or more parameters, and the manipulation data, where the 3D model of the patient is visualized in a 3D space using a plurality of graphics rendering mechanisms; generating a set of instructions for a printer based on the 3D model, and controlling the printer for generating a 3D printed model of the anatomical structure based on the set of instructions, where the 3D printed model is configured to receive an implant, such that the implant is contoured to match the fracture site of the anatomical structure.

Other and further aspects and features of the present application would be evident from reading the following detailed description of the embodiments, which are intended to illustrate, not limit, the present application.

### BRIEF DESCRIPTION OF THE DRAWINGS

Specific examples have been chosen for purposes of illustration and description, and are shown in the accompanying drawings, forming a part of the specification.
**FIG. 1** illustrates one example of a portable workstation in accordance with the disclosed technology.
**FIG. 2** illustrates one example of a workstation in accordance with the disclosed technology.
**FIG. 3** illustrates one example of a multi-user AR workstation in accordance with the disclosed technology.
**FIG. 4** illustrates a block diagram of a software and hardware architecture for the workstations illustrated in **FIGS. 1****,** **2** and **3****.**
**FIG. 5** illustrates a process according to a disclosed embodiment.
**FIG. 6** illustrates details of a rendering stage used in the process illustrated in **FIG. 5****.**
**FIG. 7** illustrates an example configuration of a computer or computing device suitable for use in the workstations illustrated in **FIGS. 1****,** **2** and **3****.**
**FIGS. 8A-8C** are schematics of network environments implementing exemplary systems for generating patient-specific implants for anatomical structures of patients, according to embodiments of the present application.
**FIG. 9** illustrates an exemplary system for generating patient-specific implants for anatomical structures of patients, according to an embodiment of the present application.
**FIGS. 10A-10B** illustrate an exemplary anatomical structure having a fracture site being manipulated using the system of **FIG. 9****,** according to an embodiment of the present application.
**FIG. 11** is a flowchart illustrating an exemplary method for generating patient-specific implants for anatomical structures of patients, according to an embodiment of the present application.

### DETAILED DESCRIPTION

Embodiments are disclosed in the context of generating patient-specific implants for anatomical structures of a patient. However, in general, the embodiments may be implemented in or for any medical or cosmetic procedure using a computing device for (i) medical imaging of a patient anatomy, (ii) visualization of an anatomical structure or site within the body of a patient using AR/VR/MR technologies, (iii) manipulation of an anatomical structure or site in a virtual environment, and/or (iv) reduction in a region of discontinuity (or fracture site) in or proximate to an anatomical structure such as a bone, tissue, organ, and any other biological element.

The presented technology relates to devices and systems for multidimensional data visualization and interaction in an augmented reality, virtual reality, or mixed reality environment. The disclosed embodiments generally apply to volumes of all sources, including but not limited to a medical imaging, an industrial computed tomography scanning, a three-dimensional (3D) or two-dimensional (2D) mathematical modeling, 3D or 2D scientific dataset, and the like. In a medical environment, the disclosed embodiments provide a tool for a doctor, physician, or other medical technician to quickly load and review patient scans in an AR/VR/MR environment. Moreover, unique, and unobstructed views of the patient's scans are easily obtainable. The physician or technician may manage a scan as if it were a physical object in a palm of his/her hand, observing it from any angle to get the best understanding of the situation at any zoom and detail level desired.

In an augmented reality configuration, the presented technology allows a user to see computer-generated 3D images overlaid in the real world, positioned correctly in relation to real objects of significance, such as parts of a patient's body or a surgeon's tools. Often, but not always, the rendered objects are identical to these features and are placed exactly where the real features are, so that the rendered versions are visible when the real objects are occluded, usually by skin or other anatomy. In a desired embodiment, the augmented reality applications of the presented technology have particular value for minimally invasive surgeries where almost the entire surgery happens underneath the patient's skin without direct visibility. Rendered images may also be purely informational. For example, it may highlight a line around a bone exactly where it needs to be cut, or draw a line along the vector through which a surgical instrument must be inserted.

In a medical environment, the disclosed embodiments generally apply to the presentation of one or multiple 3D medical imaging scans of a plurality of modalities, including but not limited to a computed tomography (CT) technique, a magnetic resonance imaging (MRI) technique, a CT angiography (CTA) technique, a MR angiography (MRA) technique, a Cone Beam CT (CBCT) technique, etc., and their post processing results. The CT technique may be a computerized x-ray imaging procedure that produces signals processed by computer to generate cross-sectional images of the body. The MRI technique may be a non-invasive imaging procedure that uses strong magnetic fields, magnetic field gradients and radio waves to produce 3D detailed anatomical images. The CTA technique may be a procedure that applies the CT technique on a patient with an injection of a special dye to produce pictures of blood vessels and tissues in the body. The MRA technique may be a technique based on the MRI technique and the injection of contrast agents for studying arterial and venous systems. The CBCT technique may be a variant of the computed tomography (CT), and is used particularly in dental and extremity imaging.

In a medical environment, the disclosed embodiments further apply to one or multiple medical imaging scans with other multidimensional data, including but not limited to a digital radiography (DR) technique, an ultrasonography technique, and their post processing results. The DR technique may be a procedure that utilize a wide beam of x-rays for 2D image acquisition of a patient. The ultrasonography technique may be a procedure that uses high frequency broadband sound waves that may be reflected by anatomy tissue to produce images of internal structures of the body.

In a medical environment, the disclosed embodiments may apply to all medical specialties that utilize the medical imaging, including but not limited to radiology, orthopedic surgery, craniomaxillofacial surgery, dentistry, cardiothoracic surgery, neurosurgery, neurology, spine surgery, otorhinolaryngology, general surgery, internal medicine, etc.

The disclosed embodiments utilize geometric primitives referred to herein as "voxels", arranged in a "volume". It should be appreciated that the precise appearance or mathematical definition of the voxels varies by application and is not essential to practice the embodiments disclosed herein. These geometric primitives include that: each primitive may be uniquely identified by 3 integral coordinates (x, y, z); the (x, y, z) values of the primitives' integral coordinates have a finite range; each primitive may be geometrically contained within a pyramid with a regular cuboidal bounding extent; the regular cuboidal bounding extents of no two primitives intersect in three dimensions; and the (x, y, z) lengths of the regular cuboidal bounding extents do not differ between any two primitives.

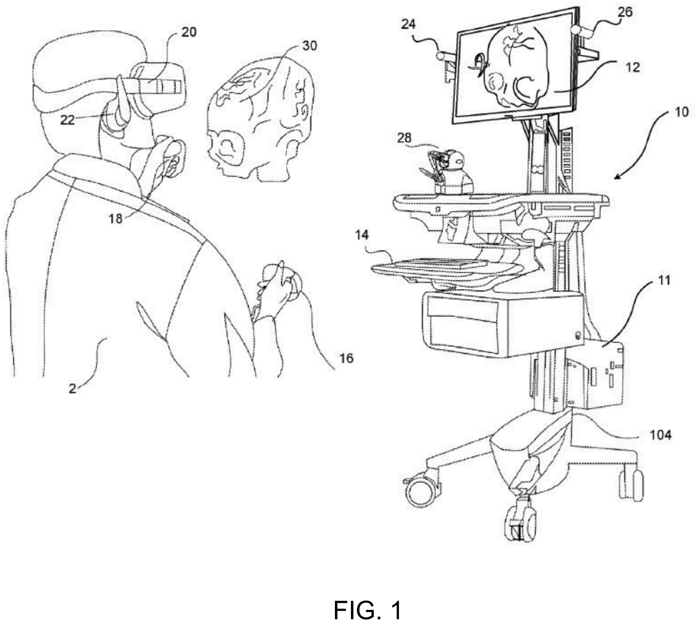

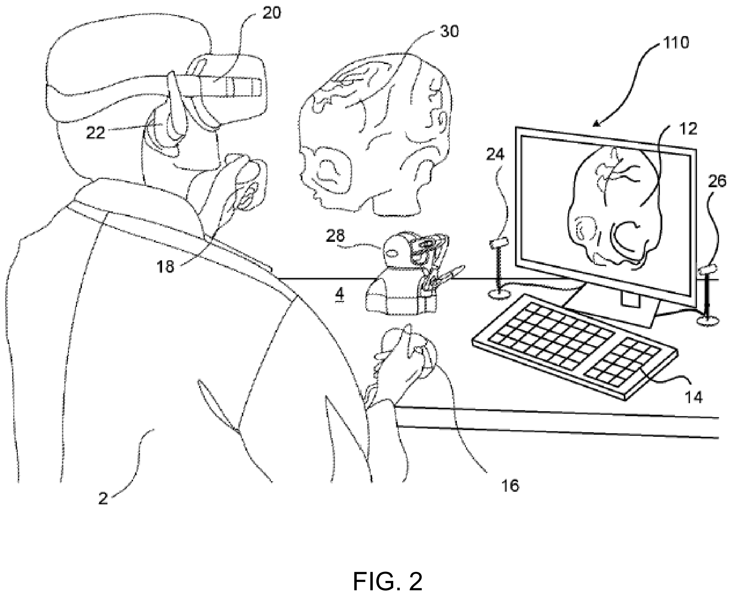

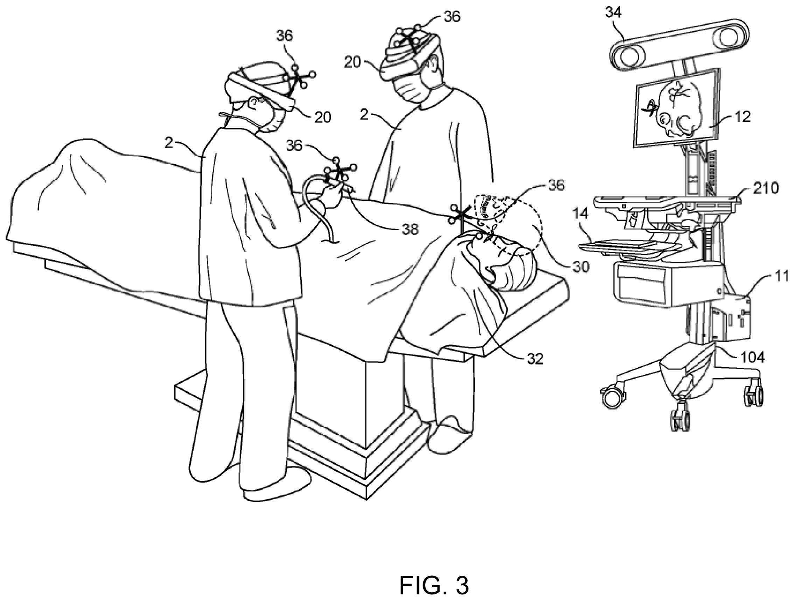

**FIG. 1** illustrates one example of a portable workstation 10 in accordance with the disclosed technology; **FIG. 2** illustrates one example of a workstation 110 in accordance with the disclosed technology while **FIG. 3** illustrates one example of a workstation 210 with an Augmented Reality configuration of the disclosed technology. In the illustrated examples, the workstations 10, 110 and 210 may include one or more AR/VR/MR devices and/or one or more haptic devices, which along with the data visualization and interaction discussed herein provide several advantages previously unattainable in the art. In one or more embodiments, the workstations may not include the haptic device. Thus, the disclosed principles and advantages over the current state of the art are obtainable on workstations 10, 110 and 210 that may not have the one or more haptic devices.

Referring to **FIG. 1****,** the workstation 10 includes a computer 11, a display screen 12, a set of keyboard and mouse 14. The workstation 10 is shown being provided on a compact wheeled carrier 104, making the workstation 10 easily transportable. The workstation 10 is shown being operated by a user 2. In the illustrated example, the user 2 is wearing an AR/VR headset 20 for viewing a stereoscopic visualization 30 of one or more sets of 3D volume data or 2D image data input into the workstation 10 and rendered by the workstation 10 in accordance with the disclosed principles. In the illustrated embodiment, a rendering of one or more 3D volume datasets or 2D image datasets in accordance with the disclosed principles may also be presented on the display screen 12.

The AR/VR headset 20 helps in viewing one or more anatomical structures by the visualization 30 of one or more sets of 3D volume data or 2D image data. The visualization 30 may be in 2D or in 3D and may be viewed from different angles and positions. The visualization 30 of one or more anatomical structures may be projected onto the actual patient which the data was previously scanned from. The visualization 30 may be superimposed with the corresponding actual anatomical structures by collocating the 3D volume data or the 2D image data with the patient body.

The AR/VR headset 20 may be connected to the workstations 10, 110 for receiving and conveying the data. Said connection may be achieved by one or more universal serial buses (USB) or display cables. The connection may also be established network connections between the workstations 10, 110 and the AR/VR headsets 20, which have standalone computation and communication capabilities. The network connection may be a local area network such as Wi-Fi network, or a high speed and low latency wide area network such as 5G cellular network or fiber broadband network.

The user 2 is also shown wearing a headphone 22 for listening to auditory simulations as the user 2 observes and interacts with the volume or the image data input into the workstation 10. In the illustrated example, the user 2 is operating two hand controllers 16, 18 also used to interact with the data rendered by the workstation 10 in accordance with the disclosed principles.

Referring to **FIG. 2****,** the workstation 110 includes a computer (not shown), a display screen 12, a set of keyboards and mouse 14. The workstation 110 is shown being operated by the user 2 at a desk 4 or other workspaces. As explained below in more detail, due to the novel multidimensional data visualization and interaction provided by the disclosed principles, patient-specific data is easily loaded into, rendered, and interacted with in both workstations 10, 110 making the workstations 10, 110 suitable for viewing critical scan information in all areas of health care facilities, including but not limited to a radiology lab, an operating room, an emergency room or a doctor's office. The workstations 10, 110 may also be suitable for patient education and engagement. Patients may be able to better understand their condition and physicians may walk the patients through proposed interventions, actively consulting with them to determine the best therapeutic choice for their respective situations. This may reduce patient anxiety and reinforce the patients understanding of their treatment and increase informed consent for medical plans of action.

Referring to **FIG. 3****,** the workstation 210 includes a computer 11, such as a desktop PC or laptop, an optical tracking device 34 connected to the computer 11 for reporting positions of real objects of significance, and a network router (not shown) for connecting the computer 11 with one or more AR headsets 20 and other devices as needed, such as the optical tracking device 34. Other input and output devices, such as the display screen 12 and the keyboard and mouse 14 are available for the user 2 to interact with the workstation 210 via graphics user interfaces (GUI). Some examples may include the workstation 210 operating in communication with a remote device. Examples of the remote device may include, but are not limited to, a handling device (such as a print head controller and a marking engine), a storage medium, a computing device, and a printer. The remote device may be configured to receive, store, process, display, email, and/or print images and 2D or 3D software models.

In an embodiment, the optical tracking device 34 may be a separate equipment that is physically connected with the computer 11. In another embodiment, the optical tracking device 34 may be a software component that receives optical input data from existing optical sensors on the AR headset 20 or any other devices of the workstation 210. The optical marker objects 36 are designed to work with the optical tracking device 34 and they may be of different modalities based on the visual fiducial system used by the optical tracking device 34. In a desired embodiment, the optical marker object 36 may be a constellation of spheres or other geometric primitives, a two-dimensional bar code such as a QR code, AR code, a constellation of two-dimensional (2D) bar codes, a 2D image, or a three-dimensional (3D) object.

The optical marker objects 36 may be attached to one or more real objects of significance and one or more AR headsets 20 to track their real time position and orientation. In the illustrated embodiment, the real objects of significance may be the patient anatomy 32 or one or more surgical instruments 38. Custom attachments are used to connect the markers to objects of significance. In a medical environment, the attachments may be custom-designed, 3D printed objects that attach to a fixture on the patient body, or directly to the patient anatomy 32 which may be the bone. There may also be such attachments on the AR headsets 20.

The workstation 210 is shown being operated by one or more users 2. In the illustrated example of **FIG. 3****,** each user is wearing an AR headset 20, which is a transparent visor with an onboard computer that allows the user to see 3D images overlaid over the real world. The AR headset 20 presents to the user 2 a stereoscopic visualization 30 of one or more 3D objects or 2D images. The optical tracking device 34 reports the position of all the markers that it sees to the workstation 210. The offset between each optical marker object 36 and the object of significance to which the optical marker object 36 may be attached is known ahead of time, based on the shape of the attachment. Similarly, the view perspective of the user 2 may be computed based on the tracked position and orientation of the AR headset 20 as well as the offset between the headset and user 2's both eyes. The workstation 210 uses these offsets to draw each rendered object in the correct location from the view perspective of the user 2, allowing the stereoscopic visualization 30 of the 3D objects or 2D images to be superimposed to their real-world counterparts, which may be the patient anatomy 32 or one or more surgical instruments 38.

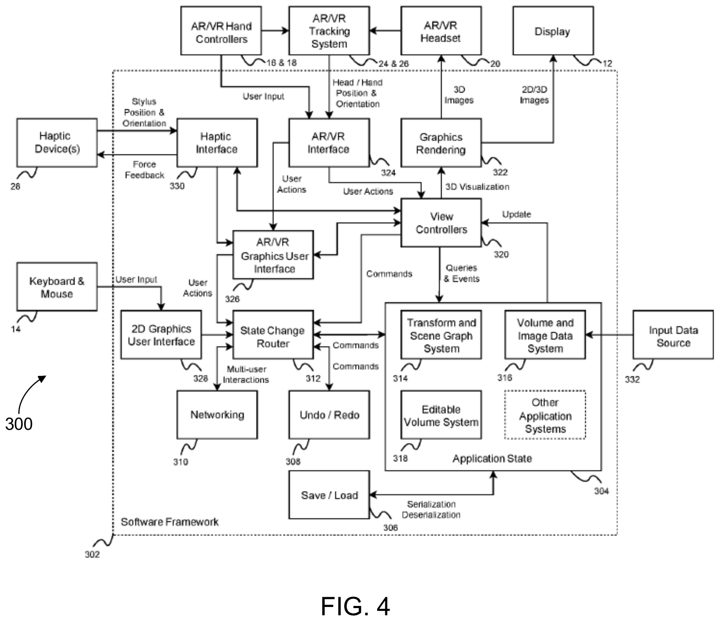

**FIG. 4** illustrates a block diagram of a software and hardware architecture 300 for the workstations illustrated in **FIGS. 1****,** **2** and **3****.** In the illustrated example, the architecture 300 includes interconnected devices and logic that are integrated by a software framework 302. The software framework 302 includes an Application State module 304 which maintains one or more virtual scenes. Each virtual scene may be a distinct state of the application that contains all data and content presented in AR/VR/MR. The Application State module 304 further comprises one or more application systems and system data. Each application system has corresponding system data, and each system data has a corresponding application system. The Application State module 304 maintains all application systems and the system data associated therewith for lifetime of the application. The Application State module 304 allows querying and interaction with any of the application system and the system data associated therewith in a specific and controlled manner. Each application system includes logic for creating, modifying, and destroying the corresponding data and serves as a target for all application commands. The application system also includes a public interface that allows querying current events and subscribing to an event that is called whenever the system data is created, modified, or destroyed. The changes made in the data may be preserved even after the user 2 leaves the scene.

The application systems comprised in the Application State module 304 may include a transform and scene graph system 314, a volume and image data system 316, a view object system 318 and plurality of other application systems that define application-specific features and user interactions.

The software framework 302 further comprises a Save/Load module 306 for saving and loading operations. The saving operation serializes all application systems and the system data associated therewith in the Application State module 304 of an active session, including one or more original, processed, or edited volume or image data, their surface representation, as well as results of the user interactions, and saves into external files. The loading feature loads complete set of data from a file, deserializes the data and then initializes the Application State module 304 as well as all relevant application systems and system data. In a desired embodiment, saved data may be saved in a portfolio of files with a unique file extension, so the loading process can identify the files by such file extension.

The Save/Load module 306 further comprises a feature that converts the surface representation of the original, processed, or edited volume or image data into polygon mesh model files and save them into the file system. In one embodiment, the polygon mesh models may be saved as STL files, OBJ files, or 3MF files.

The software framework 302 includes a State Change Router 312 that serves as a hub of the application commands. Application commands may describe the creation, modification or destruction of the system data corresponding to one or more application systems. Application commands may be received from the user interactions through AR/ VR GUI 326 or 2D GUI 328, or from command issuers, which may be View Controllers 320, an Undo/Redo module 308, or a Networking module 310. Upon receiving the commands, the State Change Router 312 further sends them to command listeners, which may be plurality of application systems in the Application State module 304, the Undo/Redo module 308, or the Networking module 310 and/or a 3D Printing module 334.

The software framework 302 further comprises the Undo/Redo module 308 for undo and redo operations. The Undo/Redo module 308 receives new application commands from the State Change Router 312 and stores the commands in a command stack for undo and redo operations. The undo operation reverses the user interaction and recovers the system data at a previous state; the redo operation reverses an undo operation and recovers the system data at a state prior to the undo operation.

Features and operations in the plurality of application systems are implemented by performing a plurality of low-level operations on the system data. To group low level operations into a single logical high-level operation, all tools perform the operations on a context object which may be first acquired from the State Change Router 312. This also serves as a locking mechanism to prevent multiple tools from modifying the one or more system data in unpredictable ways.

Each low-level operation may be implemented as a command that records the before and after states of its execution. When a tool performs operations, the context records a list of all the commands that have been executed by the current tool. When the tool is finished making changes, it releases the context to finalize its changes. The context bundles the low-level operations into a single high-level undo/redo operation so that when the user 2 triggers the undo feature, all changes made by the last tool will be reverted, even if they consist of multiple sub-commands. Once the high-level undo/redo command is assembled, it is added to a stack of previous undo-redo commands. Operations may be undone and redone by applying the appropriate state from each command. The undo/redo stack can also be serialized and saved to disk, both to support resuming a session, but additionally as a record of all the steps taken in the planning session.

The software framework 302 further comprises the Networking module 310 which supports multi-user interaction and collaboration over the network. The Networking module 310 sends the multi-user interaction data or commands from the State Change Router 312 to other users on a network. The Networking module 310 also receives the interaction data from the other users and sends it to the State Change Router 312 to modify the data held by the Application State module 304 on behalf of a remote user. In some examples, the Networking module 310 may operate in communication with the remote device over a network, such as network 802.

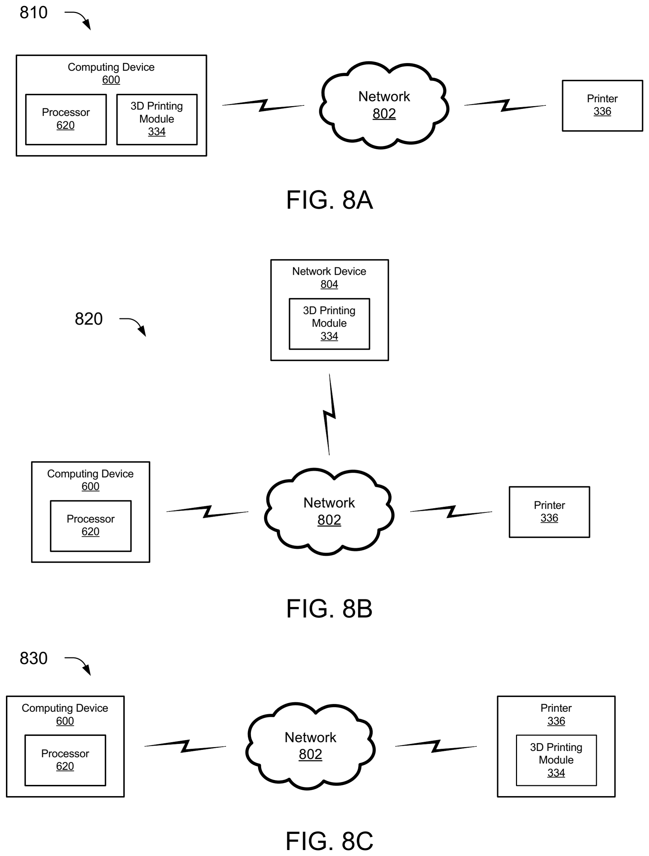

The Networking module 310 may allow multiple users to share and synchronize the entire Application State module 304, all application systems and the system data associated therewith, as well as the undo/redo stack, so multiple users may interact with the same volume objects in their own AR/VR/MR environment. Any user may be able to view and interact with one or more volume or image data, and see the changes made by others applied locally. In one embodiment, a voice/chat feature may be provided to allow users to communicate directly. The network connection may be over a local area network or a wide area network such as the Internet. The Networking module 310 may operate in communication with the 3D Printing module 334 (shown in **FIGS. 8A-8C****).**

In the illustrated embodiment, the software framework 302 includes a plurality of View Controllers 320 for visualizing the system data to the user 2 as a plurality of 3D objects, 2D objects, or graphical user interfaces (GUIs) and giving the user means to interact with the 3D/2D objects and their underlying data. The plurality of View Controllers 320 is in place for querying the public interface of the Application State module 304 for the state of one or more application systems, subscribing to events that will trigger if the data changes, and issuing the commands to create, modify, or destroy the system data based on the user instruction with plurality of interaction features. The plurality of View Controllers 320 issue the commands through the State Change Router 312. In an embodiment, the plurality of View Controllers 320 may send the command in a direct mode or an indirect mode through the AR/VR GUI 326 to the State Change Router 312.

In the illustrated embodiment, the software framework 302 may include the AR/VR interface 324 for interfacing with the AR/VR hardware, including one or more AR/ VR tracking system 24, 26, AR/VR headsets 20 and hand controllers 16, 18 worn or operated by the user 2. The AR/VR interface 324 receives the positions and orientations of user's head or hands, as well as all user inputs from AR/ VR hand controllers 16, 18. In a desired embodiment, the AR/ VR tracking system 24, 26 may track the pose of user 2's hands and further recognize hand gestures, which may trigger user actions. Said user inputs and actions are used to update the AR/VR GUI 326 and interact with plurality of View Controllers 320.

The software framework 302 further includes the graphics rendering module 322 which renders the visualization of the system data from all application systems as images captured from one or more specific viewpoints. The graphics rendering module 322 receives the system data through the plurality of View Controllers 320 and visualizes the data on one or more 2D planes or in a 3D space via plurality of graphics rendering mechanisms.

The graphics rendering module 322 may provide plurality of camera nodes that compute the correct viewer-centered perspective projection on virtual projection planes. In an embodiment, the graphics rendering module 322 may stereographically render a view of the Application State on the AR/VR headset 20. The rendering perspective may be consistent with a physical position and an orientation of the AR/VR headset 20. The graphics rendering module 322 may properly render both left and right views according to the position and orientation of the user's head given by the AR/VR interface 324. The graphics rendering module 322 is performed by the graphics processing units; the rendering results may be presented on the display screen 12 and the AR/VR headset 20 and may be reproduced on the 2D GUI 328 or the AR/VR GUI 326, which may be presented on the display screen 12 and the AR/VR headset 20.

The graphics rendering module 322 further includes a remote rendering technique which uses the computer 11 of the workstation 210 to render the images based on the view perspective of the AR/VR headset 20, and send them to the headset 20 to draw in front of the user's eyes. With a more powerful graphics processing unit and larger memory, the remote rendering technique may provide images of far greater 3D resolution and visual fidelity than is typically possible with modern standalone AR/VR headsets 20, which have limited computational power onboard (roughly equivalent of a modem cell phone).

A common challenge in all applications of remote rendering is the latency between a user performing an action and having it represented in the images. In the case of the presented technology, the displayed image is dependent on the position and orientation of the user's head, but it takes time to render the image, send it to the headset 20, and display it, while the user's head may have minor though constant movement and rotation. The nature of augmented reality makes it more important than usual to solve the issue, as the latency can be disorienting. For example, if the user was looking at a rendered object and steps to the left, the object would appear to be stuck to their head and move left with them for a brief moment, before sliding back where it belongs.

To solve this, the graphics rendering module 322 further implements a depth-based spatial latency compensation system, similar to the asynchronous space warp techniques used in spatial computing. When the workstation renders the 3D images, it also renders a "depth map," or a texture that stores the distance of every pixel in the images to the camera. This is sent to the headset 20 along with the color images. The headset 20 then draws the images on a plane, then uses tessellation to efficiently split the plane into many very small pieces, and slides each piece backwards based on the values from the depth map. This recreates the 3D shape quite closely from the rendered object viewed from the front, but importantly, user can view it from slightly different angles and the object still appears to be shaped correctly. The headset 20 then uses the timestamp of the image to move that plane to where the image was taken, which is where the headset 20 was a few milliseconds prior. Other than some minor disocclusion artifacts, this results in an up-to-date version of the old image from the new angle.

In addition, the graphics rendering module 322 may further compress and decompress the stream of images to minimize the time it takes to send the images from the workstation 210 to the AR headset 20 over the network. Both devices handle the compression/decompression using dedicated hardware to minimize latency. In one embodiment, the hardware acceleration of the Advanced Video Coding, which is also referred to as H.264, is implemented on both the workstation 210 and the AR headset 20.

The software framework 302 further incudes one or more graphics user interface (GUI) to facilitate the user 2 to interact with the software. In the illustrated embodiment, the AR/VR GUI 326 is presented in the virtual scene and interactable via AR/VR headsets 20, AR/VR hand controllers 16 & 18, user's hand movements, gestures, or user's eye movements. The illustrated embodiment further includes one or more 2D GUIs 326 that are presented on the display devices 12, and interactable via keyboard and mouse 14, or the touchscreen. User interactions via the GUIs 326 may trigger commands which change the state of the application.

The software framework 302 further includes the haptic interface 330 for mediating communication between the user 2 and the computer 11, monitoring the position, orientation, velocity, and acceleration of the mechanical stylus of the one or more haptic devices 28, and applying force feedback to the user's hands via the one or more haptic devices 28. The haptic interface 330 generates force output directly to simulate a field of force or other mechanical effects such as gravity, friction, damping, or vibration.

The haptic interface 330 sends the input data from one or more haptic devices 28 to the AR/VR GUI 326 and/or the plurality of View Controllers 320. The haptic interface 330 links the one or more haptic devices 28 with the virtual tool that further drives the plurality of View Controllers 320 to interact with the Application State module 304 and modifies the system data. The haptic interface 330 may also indirectly interact with the plurality of View Controllers 320 through the AR/VR GUI 326.

In one or more embodiments in which the one or more haptic devices 28 are being used, the graphics and the haptics may be on two separate threads. The haptics and the graphics may have distinct update schedules; for example, haptics at 1000 Hz and graphics at 90 Hz. In this example, the software framework 302 may synchronize the two consecutive graphics updates after approximately every 30 haptic updates, and it is within the skill of artisans to modify the way the haptics and the graphics update and synchronize.

The graphics rendering module 322 may implement a variety of visualizations of the volume or image data either on a 2D plane and/or in a 3D space, including but not limited to a plurality of shaded surface display (SSD) techniques, a plurality of volume rendering techniques (VRT), a plurality of multi-planar reconstruction (MPR) techniques, and a plurality of intensity projection techniques such as the maximum intensity projection (MIP) technique.

In one embodiment, the graphics rendering module 322 may implement the visualization via a plurality of shaded surface display (SSD) techniques which reflect the structures of interests by visualizing the surface representation of a volume layer generated by the volume meshing process. The volume layer is a set of geometry that shares the same rendering material and source. It may be constructed either from an iso-surface contained in a scalar volume dataset, a signed distance field (SDF) of an editable volume object, or a binary volume dataset derived from volume segmentation. Multiple iso-surface layers may be created from the same volume dataset.

The rendering of layers as geometry allows seamless multi-modality rendering. Segmentation or iso-surface layers can be mixed and matched from different scan modalities. The layers from every loaded medical imaging dataset faithfully represent the patient specific anatomy in virtual reality; they can also be accurately superimposed with the actual patient in an augmented or mixed reality environment. As an editable volume object is modified, the associated surface representation may be updated in real-time.

The graphics rendering module 322 may also implement an order-independent transparency (OIT) method which may be used to render an arbitrary unsorted polygons with correctly sorted transparency. This allows displaying the multiple volume layers and other 3D or 2D geometries with adjustable and correct transparency. Applying the OIT method, the opacity of each layer or geometry can be adjusted independently from fully opaque to fully hidden or anywhere in between. In a desired embodiment, the OIT method is implemented using an A-Buffer technique with a per-pixel linked list. As the anatomy is being rendered, the fragments are accumulated in these lists instead of directly composited to a frame buffer. At the end of a frame, the lists are sorted by depth, blended, and then composited with an opaque part of the scene.

It should be appreciated that a plurality of rendering features may be available. At both design time and runtime, the rendering features may be toggled on or off, or have their parameters changed. These features may include, but are not limited to, per-layer colors and transparencies, photo-realistic rendering, diagrammatic cutaways, soft deformation in response to touch, and X-ray visual simulation. In one embodiment, two lighting options may be available: a point light without distance attenuation attached to a camera, and an image-based lighting scheme with directional occlusion. In addition, the meshes may be exported for use in an external software.

The display outputs, from both the 3D and 2D renderings, may be presented on both the AR/VR headsets 20, and the regular computer displays 12 such as monitors, projectors, or televisions. To generate the display outputs on the AR/VR headsets 20, two scene cameras are set to move and rotate based on the positions and orientations of user's head, as well as the Inter Pupillary Distance (IPD) of user's eyes. Stereoscopic vision and depth perception are therefore achieved via the difference of the display outputs for both eyes. On regular computer displays 12, the display output can either be the clone of one of the outputs to the AR/VR headsets 20; optionally, for better experience of the surrounding audiences, the output can be obtained from a separated scene camera which may stay at a fixed point in space, or follow the perspective of the user 2 while keeping the camera movement smooth and steady.

In a desired embodiment, the plurality of volume rendering techniques may include a novel rendering technique referred to herein as a view-ray-ordered volume rendering technique. For visualization of end-user provided volume data, the workflow may be as follows: First, unnecessary structures are eliminated. To do so, the user 2 outlines a 2D region of interest on a maximum intensity projection image of the volume data about any voxel-aligned axis. This 2D region is projected into a 3D polyhedron constrained by the AABB of the one or more volume object, and any information outside of the 3D polyhedron is discarded. Next, a transfer function is specified, aided by an interactive 3D visualization. The transfer function includes one or more iso-values defining the iso-surface of interest, as selected on a data-value histogram of the one or more volumes. The transfer function furthermore includes scale and bias values that modulate a gradient magnitude driven color ramp. The color ramp tends to distinguish softer versus harder materials. Finally, opacities corresponding to the two extrema of a color ramp may be modified and rendered with exact transparency. All transfer function changes reflect immediately on the 3D rendering. Details are rendered with sub-voxel interpolated details.

The plurality of volume rendering techniques may also include a direct volume ray-caster technique, which renders multiple iso-surfaces, or an SDF obtained from the volume data by marching the ray though the one or more volume object and evaluating intersections with the surfaces. It supports multiple iso-surfaces at different scalar values, with correct transparency, and optionally participating medium rendering. Participating medium rendering simulates increasing opacity as the material gets thicker. Each surface can have different material settings, which may include but not limited to color, opacity, and density for the internal material.

The graphics rendering module 322 may also implement a plurality of MPR techniques to reconstruct a visualization of one or more volume datasets on one or more intersecting 2D planes. The scalar value at each pixel of the plane can be determined by trilinear interpolation of the voxel values of the containing voxel cell in a volume grid. The MPR can be rendered in greyscale or pseudo color with fully configurable mapping of the colors with the voxel values. Transparency can be set along with the color mapping to allow viewing of the 3D rendering behind the MPR overlay, or making certain portion, such as the space outside of the region of interest, less noticeable or even invisible.

The graphics rendering module 322 may also implement a plurality of intensity projection techniques to visualize one or more volume datasets on a 2D plane by projecting all voxels of the volume datasets into a single 2D image. Each pixel of this 2D image is a combination of all projected voxels. According to different methods by which the projected voxels are combined, the plurality of intensity projection techniques may comprise a maximum intensity projection (MIP) technique, a minimum intensity projection technique, an average intensity projection technique, a median intensity projection technique, a standard deviation intensity projection technique, and a cumulative intensity projection technique.

As discussed above, in one or more embodiments in which the one or more haptic devices are being used, the haptic interface 330 may allow interactions between the virtual tool corresponding to the one or more haptic devices 28 and elements within the virtual scene. A haptic proxy is maintained to describe the position of a haptic interface point, which tends to move towards the actual position of the haptic stylus while always staying outside of any haptic-enabled objects. Each object may be assigned with different haptic materials, including but not limited to stiffness, viscosity, static friction, and dynamic friction, as well as a plurality of physical properties such as density, gravity, elasticity, damping, etc. Therefore, the user 2 may perceive a life-like tactile feedback on different surfaces and textures when touching haptic-enabled objects.

In one or more embodiments in which the one or more haptic devices 28 are being used, the haptic interface 330 may track the events of haptic interaction, including the beginning of contact, the end of contact, continuous contact, penetration, to name a few. Custom behavior may be programmed when the events are triggered. The haptic-enabled objects may be configured to be penetrable, and the objects may be penetrated through when the force user applies to the surface of the objects exceeds a predetermined threshold.

In one or more embodiments in which the one or more haptic devices 28 are being used, the haptic interface 330 may implement one or more spatial constraints to the haptic interaction point, which may limit the DOF of the translation and/or rotation of the virtual stylus. The haptic interface 330 may also implement programmable custom haptic force effects, including but not limited to a constant force, a viscosity effect, a vibration effect, or a magnetic effect.

In accordance with the disclosed principles, and in one or more embodiments in which the one or more haptic devices 28 are being used, the architecture 300 may support, via the haptic interface 330, the haptics interaction with volume layers, which may allow the user 2 to touch and interact with one or more volume layers via one or more haptic devices 28. For each volume layer, a subset of voxels near the moving path of the haptic proxy may be collected. An iso-surface within this subset of voxels may be computed and used to determine a new position for the haptic proxy. Multiple iterations of this process may be executed within the frame to refine the proxy position. Based on the offset between haptic proxy and the actual stylus position, as well as all haptic properties applied to the volume layers, an output force may be calculated and applied to the one or more haptic devices 28 as the tactile feedback of the volume layers. The haptics interaction may also work with editable volume objects, whose data and surface representations may be modified in real-time to simulate the change of geometry such as drilling, cutting or augmentation.

In accordance with the disclosed principles, the AR/VR interface 324 may be designed and implemented to provide compatibility with various AR/VR hardware. Specifically, the AR/VR interface 324 may identify AR/VR hardware (i.e., the AR/VR headset 20 and the hand controllers 16, 18) upon startup of the application and may map the correct inputs and outputs for the headset 20 and the hand controllers 16, 18 being used. In a desired embodiment, world-based user interfaces and custom-built hand models may be implemented into the architecture 300 such that each user may receive a consistent experience even though different AR/VR headsets 20 or the hand controllers 16, 18 are being used. The AR/VR interface 324 may support dominant and secondary hand references, allowing the architecture 300 to switch from right-handed mode to left-handed mode at any time. In the disclosed embodiment, the user's hands may track any volume layer or any 3D/2D geometry in the virtual scene via distance tracking. The tracking does not need to be dependent on any collision bounds, allowing more accurate interaction with small objects that are in proximity.

In a desired embodiment, the AR/VR interface 324 includes the AR/VR GUI 326 designed specifically for being used in conjunction with the one or more volume layers and other 3D/2D geometries in accordance with the disclosed principles. Being anchored to the wrist may allow the virtual scene to be scaled up many times its original size and let the user 2 observe the volume layers or geometries from the inside. Icons and tags of the UI buttons may be rendered in a depth-independent manner, allowing the user 2 to see the buttons even when standing inside a solid volume layer. The AR/VR GUI 326 may also be easily moved or hidden to avoid obstructing the view.

As noted above, the Application State module 304 may comprise the transform and scene graph system 314 which maintains a data structure that holds the transformational relationships, such as translation, rotation, and scale factors, among all elements in the virtual scene. The data structure may maintain a transformation hierarchy that describes a relation of transformations of scene elements with each-other. The transform and scene graph system 314 may be organized around parent-child relationships via a tree structure with the origin of the global coordinate system being the root and each element in the virtual scene being represented as a node. The position, orientation and scale factor of each node may be defined by the transformation matrix, and the transformation matrix of a parent node is applicable to all its descendant nodes. Multiple tree structure may be simultaneously maintained by the transform and scene graph system 314 to reflect different states of the same set of system data, allowing the user 2 to view any certain state and/or compare between states. In a desired embodiment, multiple scene graphs may be organized to represent the patient anatomy at distinct phases of surgery, such as a preoperative phase, a plurality of the intraoperative phases and a postoperative phase.

The Application State module 304 may also comprise the volume and image data system 316. The volume and image data system 316 receives one or more 3D volume datasets or 2D image datasets generated or maintained by the input data source 332 which may be a medical scanner. Examples of medical scanners that may be used as the input data source 332 for characterizing the physical objects include, but are not limited to, the computed tomography (CT) scanner, the magnetic resonance imaging (MRI) scanner, the digital radiography (DR) scanner, or the ultrasound scanner, such as those typically used for obtaining the medical images. The input data source 332 may also be a database such as the Picture Archiving and Communication System (PACS), which provides economical storage and convenient access to images from multiple modalities.

The volume and image data system 316 may input the 3D volume data or 2D image data supplied in either a Digital Imaging and Communications (DICOM) format or an MHD/RAW format. The volume or image data with 16-bit and 8-bit integer values may be directly supported; other formats may be automatically converted to 16-bit. To accommodate distinct types of the input data sources, the data contents of scalar 3D volumes (such as CT or MRI scans), or 2D images (such as DR or ultrasound scans), as well as the binary volume or images from the segmentation of the scalar datasets may be processed and maintained by the volume and image data system 316.

The volume and image data system 316 may implement a volume meshing process which generates surface geometries from iso-surfaces across the one or more volume objects while sufficiently performant as to allow constant real-time alterations of the editable volume datasets and their corresponding surfaces. Based on the surface nets algorithm, it may be able to infer and generate a variety of sub-voxel geometric features from a trilinear interpolation function, including the disambiguation of what would otherwise be non-manifold portions of the surface. This is particularly evident in the visualization of a thin or a tunnel-like structure. Surface normal may also be generated for use in lighted rendering, in such a way as to automatically produce an appropriate mixture of hard edges and curved surfaces to satisfyingly represent complex edges without the appearance of undue darkness or obvious facets.

The volume and image data system 316 may also implement a topological smoothing process intended to be used in combination with the volume meshing process, which produces a smoother mesh from the one or more volume object of binary segmentation without overly deviating from the original geometry. Because the topological smoothing process takes place before regular meshing, the smoothed mesh and scalar data are self-consistent, and the system's output is fully and transparently compatible with any volume-manipulating features and can be trivially converted back into the original binary segmentation. The smoothing computation takes place partially on a Graphic Processing Unit (GPU).

The volume and image data system 316 may also implement a series of post processing algorithms of noise reduction to improve the visual fidelity of volume or image visualization. The edge and feature preserving smoothing algorithm may be executed upon the one or more volume or image datasets to suppress low-amplitude noise across all frequencies and make voxels or pixels of the same material cluster closer in a scalar value. Upon the output of the smoothing algorithm, the algorithm of small isolates culling may be executed to remove additional noise by replacing topologically isolated small fragments within the one or more 3D volume datasets or 2D image datasets with smoothed data. Upon the output of the small isolates culling algorithm, a deconvolution algorithm may be executed which simultaneously hardens edges or corners, and smooths where no edge or corner exists. Thus, the influence of a point spread function is removed, voxels or pixels of the same material cluster closer together in the scalar value, and the remaining fragments of noise become more topologically isolated. Upon the output of the deconvolution algorithm, the small isolates culling algorithm may be executed again-thus, topologically isolated small fragments that were not identified in the first execution of the algorithm may be replaced with the smooth data.

According to the disclosed principles, the number of segmented volume objects produced from a same source volume object may optionally be recombined into a single volume object having auxiliary layer ID voxels. A layer ID may be used to simulate a single object consisting of distinct, interconnected materials. In addition to, or alternatively, the segmented volume objects may be cropped to an Axis-Aligned Bounding Box (AABB) containing existent voxels, while retaining position information. In addition, or alternatively, the number of segmented volume objects produced from the same source volume object may be individually cropped to the AABB of the union of their existent voxels. In one embodiment, the segmented volume objects are converted to scalar volume objects via a topological smoothing process.

The volume and image data system 316 may also implement a volume editing process which allows the one or more editable volume objects and the one or more surface representations associated therewith to be modified in real-time or separated into the multiple independent segments. The area being edited may be specified by either the signed distance function (SDF) or a connected component labeling (CCL) process.

The signed distance function (SDF) is a mathematical function that can return the signed distance from the cut boundary to any point in the one or more volume objects. The SDF may include but is not limited to a plane, a geometric primitive which may be a cuboid or a sphere, or a manifold mesh. The editing modifies the original one or more volume objects to reflect the remaining part, and if needed, generates the additional volume objects for the newly cut segments. The region of interest for the editing, which is conservatively defined as any cuboidal area that could contain all voxels being modified, may define the size and dimension of the new volume objects. The voxel values from that area are copied from the original volume data. To construct the cut hollow surface in the original one or more volume objects and the solid surface in the new ones, the signed distance function shall be applied to every voxel in the region of interest in the original one or more volume objects, and then applied in the new one or more volume objects but with the distance sign reversed. The new signed distance value at any voxel shall be the minimum of the original value and the distance returned from the function.

In a desired embodiment, user may define one or more SDFs through auxiliary 3D shapes introduced via user interaction. In another desired embodiment, the volume cutting feature further comprises a paint to separate a mode adapted to define cut regions by gradually painting on one or more editable volume objects by a virtual paint bush of various shapes and dimensions. The area to be separated may be rendered with highlighting visual effects for the user 2 to preview the cut regions before cutting.

The connected component labeling (CCL) is a process which uniquely labels all subsets of the voxels whose represented geometries are connected. The volume editing may be achieved by breaking such connectivity with one or multiple mesh based cutting boundaries defined by the user 2. In an embodiment, the editable volume system may further utilize the CCL process adapted to detect the separation of the one or more volume objects and the surface representation associated therewith. In another embodiment, the CCL process may be adapted to detect whether a cut specified by the user 2 may successfully separate the one or more editable volume objects, and the forecast of the cutting results may be presented to the user 2 before the cut is finalized.

One or multiple new editable volume objects may be generated to describe the newly separated subsets of voxels, with the voxel values copied from the original one or more editable volume objects. To construct the newly cut surfaces resulted from user defined cuts on both the original and new editable volume objects, the values of the voxels in all voxel cells that intersect with the boundary mesh shall be modified according to the minimum distances between the voxels and the cut surfaces.

To update the 3D rendering of the editable volume objects, volume meshing may be re-executed once volume editing is completed. The user 2 may have multiple options to interact with the newly generated volume objects. These interaction features may include removal, maneuver, and various measurements.

The volume and image data system 316 may also implement a volume ray casting process, which may effectively and accurately calculate the first point where a given ray intersects with an iso-surface of a volume dataset, or a signed distance field of an editable volume object. This functionality facilitates other volume operations including ray casting and collision detection.

As noted above, the Application State module 304 may also include a view object system 318, which maintains objects in the virtual scene and provides a unified interface to support all core features including but not limited to undo, redo, save, load, and networking. Other application systems useful for application-specific visualizations and user interactions may derive from the view object system 318 to apply the unified interface and features.

As noted above, the plurality of View Controllers 320 may issue commands to create, modify or destroy the system data of different application systems. A plurality of interaction features may be implemented by specific application systems and corresponding view controllers. Said interaction features may comprise one of more of the following: 1) a spatial tracking feature; 2) a user maneuver feature; 3) a volume editing feature; 4) a measurement feature; 5) a snapshot feature; 6) a 3D visualization feature; 7) a 2D visualization and overlay feature; 8) a drawing and annotation feature; 9) a hardware placement feature; 10) an eraser feature; 11) a 3D comparison feature, 12) a co-registration feature, or 13) an AR superimposition feature. Each interaction feature is described below.

The spatial tracking feature may allow high precision tracking of the data in the Application State 304. For any tracking subject, which is typically associated with the AR/VR devices such as the AR/ VR hand controllers 16, 18 or the AR/VR headset 20, the distance to any tracked object can be calculated to help the plurality of View Controllers 320 execute the interaction features and specify the one or more elements in the virtual scene being interacted by the user 2. Events can be associated to each tracked object, and they can be automatically triggered if the distance to the tracking subjects meets the predefined criteria.

When a tracking request is made, the distance can be interpreted by plurality of mechanisms, including but not limited to a signed distance function (SDF), a global SDF, or a closest point searching. The SDF is a mathematical function which defines a geometric primitive, or a union of multiple primitives and calculates the distance to it from any given point in a 3D space. It may define or approximate the tracking distance to any virtual scene element based on its transform data maintained by the transform and scene graph system 314. The sign of the distance value may describe whether the tracking subject is inside or outside of the tracked objects. For any volume layer of a volume data, the global SDF can be computed to aid in accurate tracking. The nearest position on the volume layer is estimated using the gradient of the SDF as a direction to project that distance. If the tracking request occurs for the subject outside the volume grid, the nearest point on the boundary of the volume grid is used to locate the nearest position on the surface. For any objects that can be represented or approximated by a collection of points, such as the polygon meshes with dense vertices, the tracking distance can be determined by searching the point closest to the tracking subject and calculating the distance to such point.

In accordance with the disclosed principles, the user maneuver feature may allow the user 2 to intuitively move, rotate, or scale one or more elements in the virtual scene in lifelike ways. This feature may allow the user 2 to observe the one or more 3D geometries such as the volume layers from the outside or from inside out. Using triggers or buttons on the hand controllers 16, 18 as well as the position and the orientation of the hands obtained from the AR/VR interface 324, the corresponding View Controller 320 may generate commands to modify the translation, orientation and/or scale factor data maintained by the transform and scene graph system 314 to update the transform of one or more objects being maneuvered.

In one or more desired embodiments, when user 2 grabs with one hand by squeezing a trigger on the hand controller 16 or 18, one or more objects being maneuvered may be freely moved and rotated; when user 2 uses both hands to grab at empty space outside the objects, the objects may rotate and scale around their own geometric centers; when both hands grab inside an object, said object may be pivoted to user's both hands, and moved, rotated, and/or scaled with regards to the hand movement.

In one or more desired embodiments, the degree of freedom (DOF) of the maneuver may be constrained so the translation along one or more axes, and/or the rotation around one or more axes may be restricted to a limited range of motion, or even completely disabled. The user 2 may also define the rotational pivot. A set of gizmos may be present with the virtual scene elements to aid such maneuver with constrained DOF.

In accordance with the disclosed principles, the volume editing feature may allow the user 2 to modify one or more editable volume objects in real-time. The volume editing feature may implement a volume cutting tool, which allows the user 2 to cut the one or more editable volume objects and the surface representations associated therewith in user defined regions. When the user 2 confirms the cuts, the editable volume objects are then modified so the corresponding surface representation matches the cuts, and the additional volume objects may be generated to represent the newly cut partitions. The volume editing feature may also implement a paint-to-segment tool which allows the user 2 to define cut regions by gradually painting on the one or more volume objects by a virtual paint brush of various shapes and dimensions. The volume editing feature may also implement a volume sculpting tool which allows the user 2 to frequently modify the one or more volume objects and the surface representation associated therewith in the region specified by the user 2, to gradually remove materials from the represented geometry or add materials to it.

The measurement feature may provide accurate 3D and 2D measurements of a plurality of spatial properties based on the source dataset. An application system for the measurement feature may be implemented within the Application State module 304 to maintain and control the data that describes all measurement elements. The measurements may be one of more of the following: 1) the distance between two points, 2) the cumulative length of a polygonal chain, 3) the angle between two lines, 4) the angle between two planes, 5) the circumference of a circle, 6) the volumetric size of a user defined space, and/or 7) the volumetric size within an iso-surface. The measurements feature may further utilize a surface binding process to attach measurement points onto a surface of any volume layer or other 3D geometry close by, or onto a plane that display 2D images or renderings. As can be appreciated, this may increase the accuracy of the point placement, thus increasing measurement accuracy. When the user 2 maneuvers scene elements, the attached measurement points may be moved altogether, and the measurement results may be updated in real-time.

The snapshots feature may allow the user 2 to capture one or more pictures or videos of the virtual scene from any user specified perspective at any user defined time. One embodiment may allow the snapshot pictures to be saved as "PNG" files, and the snapshot videos to be saved as "MP4" files. The user 2 may look through a virtual viewfinder to help focus on the virtual objects to be captured. Once a snapshot is taken, a preview may be displayed on the AR/VR GUI 326, and the image may be saved under a designated path. The user 2 may switch between portrait and landscape modes as desired. Once the snapshots are saved, they may be reviewed by the user 2 on the AR/VR GUI 326, and the saved files may be accessed by the user 2 later.

The 3D visualization feature may provide real-time configurations of the visual properties of one or more 3D objects, which may be volume datasets or 3D geometries. These visual properties include but not limited to colors, level of transparency, iso-values, transfer functions, special visual effects achieved by shaders, etc. An application system may be implemented within the Application State module 304 to maintain, and control said visual properties. A graphics rendering module 322 may update the rendering of the one or more 3D objects in real-time to reflect the changes of the visual configuration.

The 2D visualization and overlay feature may present a 2D visualization of one or more 3D volume datasets or 2D image datasets in the virtual scene. A plurality of 2D rendering techniques, such as the multi-planar reconstruction (MPR) techniques, or the intensity projection techniques may be applied to visualize one or more volume datasets in 2D. In a desired embodiment wherein one or more 2D image datasets exist, the rendering of 2D dataset may also be presented. The rendering of multiple datasets may also be combined by an image fusion technique. The 2D visualization may be presented on an AR/VR GUI 326, a 2D GUI 328, or one or more 2D planes across the 3D visualization of the one or more volume datasets in the virtual scene. The planes may be the axial, sagittal, or coronal planes of the 3D volume, or they may be in any arbitrary orientation. Optionally, the 3D visualization of the volume datasets on either side of any plane may be culled out to better present both the internal structure of the volume datasets and the 2D rendering overlaid on the planes. The graphics rendering module 322 may update both the 3D visualization and the 2D rendering overlay in real-time based on the user interaction. A specific application system may be implemented within the Application State module 304 to maintain the data essential to the 2D visualization and overlay feature.

The drawing and annotation feature may allow the user 2 to draw or mark annotations in the virtual scene. One or more annotations, which may be points, lines, curves, symbols and/or texts may be created via a drawing and annotation tool controlled by the AR/ VR hand controller 16, 18. An application system for drawing and annotation may be implemented within the Application State module 304 to maintain and control said annotations. In a desired embodiment, the annotations may be applied on the surface of one or more 3D geometries such as volume layers or 3D geometries and moved along with the associated 3D objects. In a desired embodiment wherein one or more 2D rendering planes exist, the drawing and annotations may be applied on the 2D planes. The drawing feature may also include an option to measure the accumulated length of the lines or curves. Visual properties such as the color, the line width and the dash style may be configurable through the AR/VR GUI 326.

In a desired embodiment, a dynamic annotation, which may behave like a laser pointer, may be created, and controlled by the user 2 to point out specific positions and features on the one or more virtual scene elements for the benefit of the viewing audience. The point where the laser encounters the one or more surfaces or volume layers may be calculated by a ray casting technique, and the point may be visually highlighted to help draw attention to the point of interest. In a desired embodiment wherein, multiple users participate in a real-time interactive session via networking, the movement of the dynamic annotations may be synchronized with all users over the network through commands exchanged via the networking module 310.

The hardware placement feature may introduce one or more of the 3D models to the Application State module 304. These 3D objects can be independently included in the virtual scene or mounted to an existing element in the scene. In a desired embodiment wherein, the application is implemented for one or more of surgical planning, patient engagement and/or medical education, the hardware objects may be models of medical implants, surgical plates, screws, or surgical instruments. An application system for hardware placement may be implemented within the Application State module 304 to maintain and control the hardware models. In the transform and scene graph system 314 the hardware may be attached to any element in the scene.

In a desired embodiment wherein one or more 2D rendering planes exist, one or more projected contours or cross sections of one or more hardware models may be generated and superimposed on the 2D renderings of the volume or image datasets on the planes. The projected contours or cross sections may reflect the real-time position and orientation of the hardware models with respect to the 3D volumes or 2D images visualized on the corresponding 2D rendering planes. When the user 2 maneuvers one or more hardware models in the 3D space, the projected contours or the cross sections may be updated simultaneously. When the user 2 maneuvers one or more projected contours or the cross sections on 2D planes, the same movement may be applied to corresponding hardware models in real-time.

In a desired embodiment, the hardware models such as surgical plates may be bent against the surface of one or more volume layers, fitting the curvature of the anatomy structure. In another desired embodiment wherein the hardware models overlap with other 3D objects, the level of overlapping may be measured, and may be visualized by a color gradient on the surface of the objects representing the depth of intersection.

The eraser feature may allow the user 2 to erase one of more elements from the virtual scene. The eraser may be controlled by user's hand movement via the AR/VR interface 324, and the tracking feature may monitor its distance to all erasable objects in the virtual scene. When the user 2 moves the eraser onto one or more erasable objects, a specific View Controller 320 of the eraser feature may issue a command to destroy the system data corresponding to said objects, which then triggers the removal of the objects from the view.

The 3D comparison feature may allow the user 2 to view and compare one or multiple sets of 3D objects. In one embodiment, the visualization of multiple sets of volume datasets, which may be the digital twins of the patient anatomy at different surgical phases, may be placed side by side for direct comparison. In another embodiment, the visualization of multiple volume dataset may overlay with each other for better comparisons. In another embodiment, the one or more volume objects may superimpose with their own mirrored inversion, highlighting the symmetric differences.

The co-registration feature may aid in aligning (co-registering) multiple elements, such as 3D volume datasets, 2D image datasets, and 3D geometries. The datasets may be of different modalities. The co-registration represents pairwise proper rigid transforms between the coordinate spaces of the elements. The 3D volumes may be visualized by either 2D multi-planar reconstruction (MPR) on axial/sagittal/coronal planes or overlaid maximum intensity projections (MIP). The co-registration may be performed manually via the mouse and keyboard controls, or semi-automatically via a partial Procrustes superimposition of plurality sets of user designated feature points with each set specifying the same feature on different elements. A resulting transformation matrix may be computed to describe the co-registration and said transformation matrix may be applied in the transform and scene graph system 314 to align these elements in the virtual scene.

The AR superimposition feature may superimpose the visualization of the virtual scene with real-world objects and maintain constant relative rotations and translations. The user 2 or equivalently any trained professional may register one or more 3D volumes, 2D images, or 3D geometries with virtual 3D objects that represent the optical marker objects 36 in the virtual scene. The optical marker objects 36 may be attached to one or more real-world objects of significance in the same way that their digital twins are registered with the virtual scene. When an AR headset, or a VR headset with camera passthrough is in use, a plurality of optical tracking techniques may be used to detect the 3D position and orientation of the physical markers in real time, allowing their virtual counterparts to overlay with them. Following the transformational matrix maintained in the transform and scene graph system 314, the rest of the virtual scene may be correctly superimposed with real-world counterparts when displayed through the AR/VR headset 20. When one or more real-world objects with fiducial markers are moved, all corresponding virtual objects may move automatically to maintain the superimposition. In a desired embodiment, the objects registered with the fiducial markers may be surgical instruments, or the body of a patient; the fiducial markers may be one or more blocks with QR code, or one or more sets of 3D optical markers.

In a desired embodiment, one or more real-world objects, which may be anatomy structures inside of a patient body, may be partially or fully invisible due to obstructions by other objects on the outside, and the AR superimposition feature may help reveal such internal objects by visualizing the superimposed virtual counterparts. In another desired embodiment, the superimposed virtual objects may represent a state different from the real-world objects, for instance, the preoperative anatomy vs. the surgical planning; such AR superimposition may highlight the differences between multiple states and guide any actions that need to be performed, which may be surgical procedures.

In an example, the user 2, who may be a doctor, may want to view the 3D reconstructions of the head of a patient and conduct surgical planning using the workstation 10, 110. The doctor may use the workstation 10, 110 and may be wearing the AR headset 20 for viewing the 3D and 2D visualizations of the medical scans of the brain. The doctor may import the 3D medical scans as the cranial CT or MRI, the 2D medical scans such as DR, the segmentation of the medical scans and/or 3D geometries representing patient anatomy into the Application State module 304 via the input data source 332. The entirety of the Application State module 304 may also be previously saved in one or more files, which may be loaded on the workstation 10, 110 and viewed using the AR/VR headset 20.

The AR/VR interface 324 may update the doctor's hand position, the doctor's head position and orientation data from the AR/VR hardware (i.e., the AR/VR headset 20 and the hand controllers 16, 18) to the AR/VR GUI 326 or the plurality of view controllers 320. The plurality of view controllers 320 may issue one or more commands for creating, modifying, or destroying the data to the state change router 312. In turn, the state change router 312 may further route the commands to the Application State 304, the undo/redo module 308 and/or the networking module 310. When said commands are received by the Application State 304, the system data corresponding to one or more application systems may be created, modified, or destroyed; when said commands are received by the undo/redo module 308, they may be maintained in the undo/redo stack for future undo/redo operations, which may reverse previously executed commands; when said commands are received by the networking module 310, the commands may be sent to and synchronized with other users on the network. Through the execution of commands, the doctor may interact with the virtual representation of the patient head and use all available features in the application to explore the patient anatomy, conduct the surgical planning, perform the patient consultation, or assist the surgery.

The graphics rendering module 322 may render the 3D and 2D scans of the head anatomy in 3D space or on one or more 2D planes via the plurality of graphics rendering mechanisms. The graphics rendering module 322 may properly render both left and right views according to the position and orientation of the doctor's head. The images of the brain may be presented on the display screen 12 and the AR/VR headset 20.

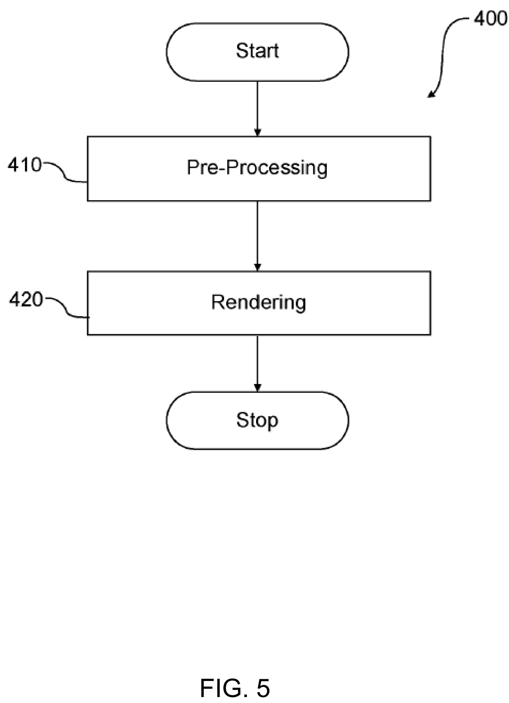

When AR superimposition is configured, the AR/VR headset 20 may augment anatomical structures of the patient head with the visualization of its digital twin. On the display of the AV/VR headset, the original image and models may be superimposed onto corresponding anatomical structures of the actual patient to reveal internal anatomy structures that are not visible from the outside; the results of surgical planning may also be superimposed onto the actual patient to help guide the surgical procedures. Virtual models and scans may move and orient correctly when the corresponding actual anatomy is viewed from different angles and positions. FIG. 5 illustrates a process 400 according to a disclosed embodiment. As discussed above, the process 400 is the view-ray-ordered volume rendering technique for the GPU.

It is well known that the view-ray-ordered volume rendering for the GPU is a computationally expensive operation. The medical images can contain hundreds of millions of voxels. Thus, it may be desirable to restrict rendering to only the interesting voxels; however, even one iso-surface of interest within the medical image may easily include tens of millions of voxels. Furthermore, for many applications, it may not be sufficient to render the outermost visible surface of the object embedded in a volume. Numerous transparent elements may be visible throughout the volume. This may prohibit a simple Z-buffering technique.

At the same time, the expectations of display resolutions and frame rate are climbing, which further exacerbate the difficulties of the volume rendering. Typical volume rendering techniques support interactive rates of approximately 10 Hz, usually restricted to a small viewport in a desktop application. On the other hand, modern users demand virtual and or augmented reality experiences, which may run at the frame rate of up to 90 Hz with render resolutions upwards of 5 million pixels. The render resolution required for the virtual or augmented reality rendering climbs higher every year. Under these circumstances, volume rendering may present an extreme computational burden that may only be met by the graphics processing unit (GPU).

Generally, volume rendering may be classified as an image-order technique or an object-order technique. The Image-order technique traces the ray or a cone through the volume for every pixel on the imaging plane. This is described e.g., in Hanspeter Pfister, "Hardware-Accelerated Volume Rendering", Visualization Handbook, 2(3), pp. 229-258, Elsevier, 2005 (hereinafter "Hanspeter Pfister"). While many techniques for accelerating these ray-traces exist, a volume ray-tracing scales poorly with increasing resolution. Small stepping of the rays leads to good locality of the reference, but the exhaustive sampling of the volume across too many pixels is inefficient. Large stepping of the rays via an empty-space-skipping technique alleviates the burden of sampling, but introduces locality of reference problems, which also results in inefficiency (particularly for complex volumes). The Image-order techniques predominate due to their simplicity of implementation, but they do not suit real-time, high-resolution applications.

The object-order techniques, on the other hand, draw the volume elements onto the imaging plane. One technique is known as a volume slicing technique, whereby the volume is rendered via an ordered stack of textured quads. This is also described in Hanspeter Pfister. While possessing some advantages, such as high locality of reference and good utilization of hardware interpolation, the volume slicing is an exhaustive sampling of every voxel in the volume. Thus, it only scales to small volumes. Furthermore, slicing exhibits serious rendering artifacts.

The other major object-order technique is a volume splatting technique, whereby each voxel is independently drawn onto the imaging plane. Described in Hanspeter Pfister. A central consideration of the splatting is how to draw the voxels in order, so that they composite together correctly. Existing techniques include an exhaustive Painter's Algorithm rendering technique from a dense 3D array, as described e.g., in G. Frieder, D. Gordon and R. A. Reynolds, "Back-to-Front Display of Voxel Based Objects", in IEEE Computer Graphics and Applications, vol. 5, no. 1, pp. 52-60, Jan. 1985; pre-sorting for one or more view directions on the CPU, as described e.g., in F. Vega-Higuera, P. Hastreiter, R. Fahlbusch and G. Greiner, "High performance volume splatting for visualization of neurovascular data", VIS 05. IEEE Visualization, 2005., 2005, pp. 271-278; or Inigo Quilez, "Volumetric Sort", (https://iquilezles.org/articles/volumesort/) (hereinafter "Volumetric Sort"); or extracting a voxel order from a multidirectional run-length encoding format as described e.g., in J. Orchard and T. Moller, "Accelerated Splatting using a 3D Adjacency Data Structure", Proceedings-Graphics Interface, 2001., 2001, pp. 191-200. All existing techniques are fundamentally sequential and CPU-bound.

Unlike the image-order and the object-order techniques, and unique solely to the view-ray-ordered volume rendering process disclosed herein, is the way the GPU is used to draw the voxels in view order, from a compressed representation of the voxel coordinates. The coordinate compression keeps memory consumption and bandwidth usage low. High locality of the reference is maintained through all stages of the algorithm.

As will be discussed below in more detail, the view-ray-ordered volume rendering process comprises a pre-processing stage 410 followed by a rendering stage 420. In the pre-processing stage 410, each voxel possibly existent within the coordinate range of the one or more volume objects must undergo a binary classification as existent or nonexistent; i.e., to be rendered or not rendered, respectively. The details of this classification are application-dependent and not essential to practice the principles disclosed herein. However, the view-ray-ordered volume rendering process performs an amount of work proportional to the number of the existent voxels (until the final rasterization stage). Thus, the view-ray-ordered volume rendering process requires the binary classification as an input. The classification is either created as, or transformed to, a binary mask volume.

In one embodiment, the binary mask volume is transformed into a Histogram Pyramid such as one described e.g., in G. Ziegler, C. Theobalt, and H.P. Seidel, "On-the-fly Point Clouds through Histogram Pyramids." // the International Fall Workshop on Vision, Modeling and Visualization. Vol. 2006, pp. 137-144. Amsterdam, The Netherlands: IOS Press, 2006. In the disclosed embodiment, the Histogram Pyramid is a hierarchical grid of element counts per grid cell. The original binary mask volume is retained to comprise the leaves of the Histogram Pyramid.

Each existent voxel may be logically assigned an index according to some fixed and particular (but arbitrary) traversal order of the Histogram Pyramid, henceforth a "fixed order." Voxel indices range from 0 to the number of existent voxels.

A look-up table may be created that maps high-order bits of the voxel coordinates to high-order bits of voxels 'fixed order' (i.e., via a prefix sum). Exactly how many bits are mapped is an implementation detail that is not essential to practice the principles disclosed herein. In any case, the binary mask volume and the look-up table may be constructed such that they are sufficient to transform any existent voxel's coordinates to its fixed order index.

As used herein, the voxel data may comprise any desired attributes for rendering each voxel, such as e.g., color, opacity, emittance, scalar magnitude, or partial derivatives of gradients. For each existent voxel, the voxel data may be stored in the fixed order. Depending on the application, the 1D fixed order may be mapped to the GPU storage directly via a buffer, or to elements in a 2D or 3D texture using a space-filling curve. In addition to, or alternatively, the voxel attributes themselves may be compressed in the 2D texture.

In the rendering stage 420, the voxels may be rasterized in order of distance from the camera. The rasterization feature of the graphics processor may be exploited. According to the disclosed principles, and without loss of generality, front-to-back compositing with front-to-back view order may be implemented herein. The rendering stage 420 is generally described as follows.

Indirect drawing may be enabled on the graphics processor. The number of vertices to draw may be supplied as the number of existent voxels. The vertex ID provided to the vertex program is interpreted as a voxel draw sequence number. Using one Histogram Pyramid traversal per vertex shader invocation, the voxel draw sequence numbers may be transformed to 3D coordinates of the existent voxels as sorted in view order. The sorted voxels may be rasterized immediately by the graphics pipeline and then discarded. Because graphics hardware guarantees correct ordered blending of the primitives according to their order of submission, this process may be sufficient for correct rendering in view order.

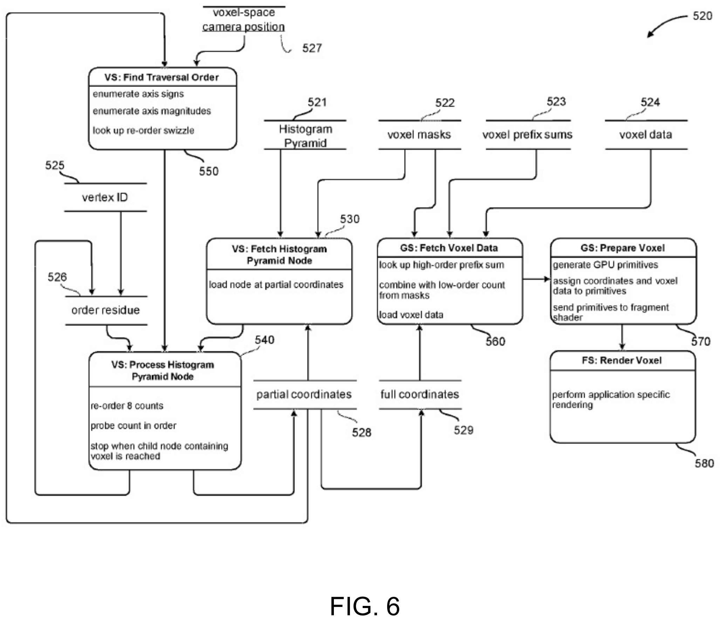

The rendering stage 420 may be implemented via a vertex shader (VS), a geometry shader (GS), and a fragment shader (FS). Details of the rendering stage 420 now follow with respect to FIG. 6. Specifically, FIG. 6 illustrates details of the rendering stage 420 that may be used in the view-ray-ordered volume rendering process 400 illustrated in FIG. 5. The rendering stage 420 is shown having six functional blocks 530, 540, 550, 560, 570, 580. The blocks 530, 540 and 550 may be implemented by the VS, the blocks 560 and 570 may be implemented by the GS, and the block 580 may be implemented by the FS. Inputs 521, 522, 523, 524, 525, 526, 527, 528 and 529 into the functional blocks 530, 540, 550, 560, 570, 580 are also shown in **FIG. 6****.**

In the illustrated embodiment, the VS implements block 530 to fetch the Histogram Pyramid node, the block 540 to process the Histogram Pyramid node and the block 550 to find the traversal order; the GS implements the block 560 to fetch the voxel data and the block 570 to prepare the voxel for rendering; and the FS implements the block 580 to render the voxel. This functionality is now described.

The VS may fetch the Histogram Pyramid node (block 530) using the Histogram Pyramid input 521 and the voxel mask input 522. At the block 530, the node may be loaded at a partial coordinates input from the partial coordinates input 528. The loaded node at the partial coordinates may be input into the block 540. The block 540 may also input the output of the block 550 (traversal order) and an order residue from the order residue input 526. In the block 540, the VS, may re-order the eight counts of the node, probes each count in order, and may stop when the child node containing the voxel is reached. In block 550, the VS may enumerate axis signs and magnitudes and look up a re-order swizzle using inputs such as the voxel-space camera position 527 and the partial coordinates 528.

For any view ray, exactly forty-eight traversal orders are possible per Histogram Pyramid node, due to the grid structure of the Histogram Pyramid. These orders may correspond to permutations of six axis orders multiplied by eight axis signs as described e.g., in Volumetric Sort. For each step of the Histogram Pyramid traversal (block 540), a traversal order may be determined (block 550) and used to rearrange the eight Histogram Pyramid node counts into an order of increasing distance with respect to the view ray. The linearized counts may then be probed until the ancestor node of the current voxel is found (block 540).

To determine the Histogram Pyramid node traversal order (block 550), the voxel-space camera position 527 may be subtracted from the bounding box center of the voxel-space Histogram Pyramid node. The resultant vector may be normalized. The signs and relative magnitudes of the normalized view vector's three components may be transformed into a discrete traversal order between 0 and 47.

For an orthographic projection, all the view-rays are identical with respect to all the Histogram Pyramid nodes. Only the voxel-space imaging plane normal may be needed to determine the traversal order, and this is constant per invocation of the VS. Therefore, upon every access to the Histogram Pyramid node, the node may be re-ordered in a constant manner.

Such constant re-ordering may be achieved via a pre-compiled shader variant per traversal order, with the correct one selected just prior to the rendering.

The voxel coordinates 529 may be passed from the VS to the GS and used at the block 560 to fetch the voxel data. The block 560 inputs the voxel masks 522, the voxel prefix sums 523 and the voxel data 524 and may perform the following functions: looks up the high-order prefix sum, combines it with the low-order count from the masks and loads the appropriate voxel data. The output of the functional block 560 is input into the block 570, which may prepare the voxel for the rendering. To do so, the block 570 may generate the GPU primitives, assigns coordinates and the voxel data to the primitives, and send the primitives to the FS.

To perform these functions, the fixed-order voxel index may be obtained via the sum of the value from the look-up table at the high-order bits of the voxel coordinates with a Hamming weight of the preceding voxel existence bits from the low-order binary mask obtained during the Histogram Pyramid traversal. The voxel data may then be fetched using the fixed-order index.

The FS performs the block 580, which is the rendering of the voxel. To do so, the FS may perform an application-specific rendering. For example, in one application, the voxel data may include scalar values for each of eight corners, and a volume rendering integral may be evaluated along the view ray passing through the fragment and the voxel. Correct ordered transparent composition may guaranteed based on the disclosed principles.

As discussed above, the execution of the rendering stage 420 may be split between the VS, GS, and FS. This may be done, in part, because it is necessary to access required GPU hardware functionality, such as primitive generation, throughout the rendering stage 420. This splitting may also suit the GPU's internal load-balancing mechanisms. The GPUs may be deeply pipelined and allow numerous VS, GS and FS invocations all running simultaneously.

In implementation, a plurality of tasks may be placed in a different type of shader. For example, the GS may perform the Histogram Pyramid traversal. This would not fundamentally alter the rendering stage 420. The described view-ray-ordered volume rendering process 400 may use the voxels as the parallel primitive for both the voxel order determination and the drawing. The existing splatting algorithms may use the voxels as the primitive only for parallel or sequential drawing. The disclosed principles, however, may allow broad data parallelism, highly coherent branches, and high data locality between nearby shader processing units. Furthermore, all data structures employed may be simple and compact enough to be fully re-generated at interactive rates.

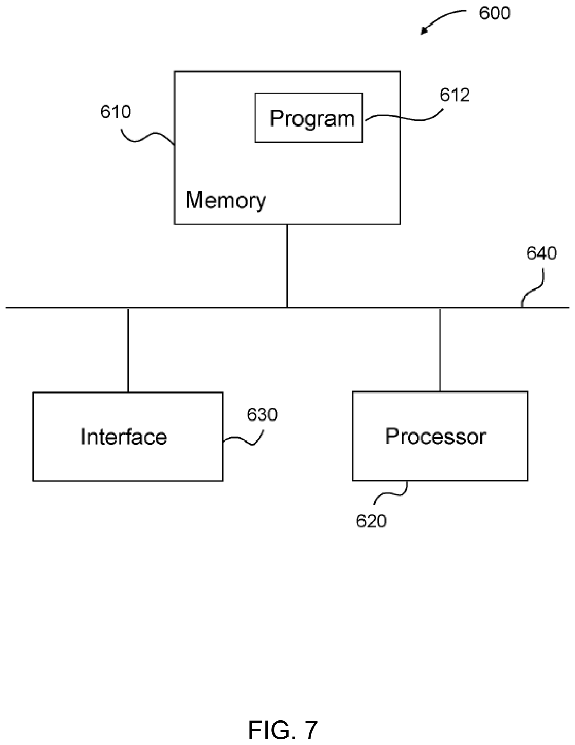

**FIG. 7** illustrates components of a computing device 600 that may be utilized in or with the workstations 10, 110, 210 to execute or that may embody components of the disclosed embodiments. For example, the computing device 600 may include a memory 610, program instructions 612, one or more processors (e.g., processor and a graphics processor) 620 to execute the program instructions 612, one or more interfaces 630 (e.g., AR/VR interface 324) connected via one or more buses 640. The memory 610 may be or include one or more of cache, RAM, ROM, SRAM, DRAM, RDRAM, EEPROM and other types of volatile or non-volatile memory capable of storing data. The one or more processors 620 may be or include multiple processors, a single threaded processor, a multi-threaded processor, a multi-core processor, or other type of processor capable of processing data.

The processors 620 may be operable to configure one or more modules, such as those mentioned above, to implement the methods and the underlying concepts disclosed herein. For example, a module may be implemented as a hardware circuit comprising custom very large-scale integration (VLSI) circuits or gate arrays, off-the-shelf semiconductors such as logic chips, transistors, or other discrete components. In another example, a module may be implemented in programmable hardware devices such as field programmable gate arrays, programmable array logic, programmable logic devices, graphics processing units, or the like. In some examples, a module may also be at least partially implemented in software for execution by various types of processors. An identified unit of executable code may, for instance, include one or more physical or logical blocks of computer instructions that may, for instance, be organized as an object, procedure, or function. Examples may include the executables of an identified module need not be physically located together or disparate instructions stored in different locations that, when joined logically together, comprise the module and achieve the stated purpose for the module. Further, the modules may be stored on a computer-readable medium, which may be, for instance, a hard disk drive, flash device, RAM, tape, and/or any other such non-transitory computer-readable medium used to store data without deviating from the scope of the subject matter disclosed herein. In a further example, a module of executable code could be a single instruction, or multiple instructions, and may even be distributed over several different code segments, among different programs, and across several memory devices. Similarly, operational data may be embodied in any suitable form and organized within any suitable type of data structure. The operational data may be collected as a single data set, or may be distributed over different locations including over different storage devices, and may exist, at least partially, merely as electronic signals on a system or network. It should be noted that one or more components of the computing device 600 may be located remotely and accessed via network. Accordingly, the system configuration provided in **FIG. 7** is provided to illustrate how embodiments may be configured and implemented.

**FIGS. 8A-8C** are schematics of network environments implementing exemplary systems for generating patient-specific implants for anatomical structures of patients, according to embodiments of the present application. As illustrated, the systems 810, 820, 830 may include the computing device 600 in communication with one or more remote devices over a network 802. The network 802 may include any software, hardware, or computer applications capable of providing a medium to exchange signals or data in any format known in the art, related art, or developed later. The network 802 may include, but is not limited to, social media platforms implemented as a website, a unified communication application, or a standalone application. Examples of the social media platforms may include, but are not limited to, Twitter^{™}, Facebook^{™}, Skype^{™}, Microsoft Lync^{™}, Cisco Webex^{™}, and WhatsApp^{™}. Further, the network 802 may include, for example, one or more of the Internet, Wide Area Networks (WANs), Local Area Networks (LANs), analog or digital wired and wireless telephone networks, Wi-Fi^{®}, radio, television, cable, satellite, and/or any other delivery or tunneling mechanism for carrying data. The network 802 may include multiple networks or sub-networks, each of which may include, e.g., a wired or wireless data pathway. The network 802 may include a circuit-switched voice network, a packet-switched data network, or any other network configurable to carry electronic communications. For example, the network 802 may include networks based on the Internet protocol (IP) or asynchronous transfer mode (ATM), and may support voice using, for example, VoIP, Voice-over-ATM, or other comparable protocols used for voice, video, and data communications.

In a first exemplary embodiment **(****FIG. 8A****),** a system 810 may include the computing device 600 in communication with a printer 336 over the network 802. The printer 336 may operate as a multifunction device having one or more print or marking engines configured to implement any of the variety of printing technologies known in the art, related art, or developed later. In one example, the printer 336 may operate as a 3D printer configured to print or generate physical objects in a 2D form and/or a 3D form. Other examples may include the printer 336 being a 2D printer configured to generate an object in 2D form or print on a 2D substrate. In this embodiment, the computing device 600 may include the 3D printing module 334 in any suitable form factor.

The 3D Printing module 334 may be installed, integrated, or operatively associated with the computing device 600. For example, the 3D Printing module 334 may be initiated or triggered by the Networking module 310. In some examples, the 3D Printing module 334 may operate in direct communication with the State Change Router 312. In some other examples, the 3D Printing module 334 may include the Networking module 310, or vice versa. The 3D Printing module 334 may be preconfigured, or dynamically configured by the processor 620, to (1) communicate synchronously or asynchronously with one or more software applications, databases, storage devices, interfaces, or appliances operating via same or different communication protocols, formats, database schemas, platforms or any combination thereof, to send and receive a variety of data; (2) collect, define, store, and analyze the data; (3) formulate one or more tasks for being performed on or trained from the data; (4) provide, execute, communicate, and/or assist in formulating one or more mathematical or computerized models for tasks related to collection, identification, manipulation, and/or presentation of the data; (5) display, print, or communicate the identified, manipulated, and/or presentable data; and (6) transfer or map the models, tasks, parameters, attributes, and associated values of the data to one or more networked computing devices and/or data repositories; (6) retrieve a virtual 3D model from a storage medium such as the memory 610 or the input data source 332; and (7) generate or fetch a set of instructions to control a printer such as the printer 336. In some examples, the 3D Printing module 334 may be further configured to generate or fetch a set of instructions to control a 2D printer. The 3D Printing module 334 may establish a connection to the printer 336.

The 3D printing module 334, in one example, may be configured to form a part of the software framework 302 discussed above. For instance, the 3D printing module 334 may be implemented as a software application or a device driver. Alternatively, the 3D printing module 334 may represent any of a wide variety of physical computing devices configured for performing various functions including those mentioned above. In some instances, the 3D printing module 334 may enhance or increase the functionality and/or capacity of the network, such as the network 802, to which it may be connected. In some other instances, the 3D printing module 334 may be also configured, for example, to perform notification tasks, security tasks, network management tasks including Internet protocol (IP) address management, and other tasks. The 3D printing module 334 in some instances may, however, include software, firmware, or other resources that support the remote administration, operation, power control, and/or maintenance of the computing device 600. The 3D printing module 334 may communicate with various modules and interfaces of the software framework 302 discussed above. Further, in some examples, the 3D printing module 334 may be configured to convert communications, which may include instructions, queries, data, files, etc., from the computing device 600 into appropriate formats to make such communications compatible with network devices (e.g., the printer 336, another computing device or workstations, etc.) and vice versa. Consequently, the 3D printing module 334 may allow implementation of network devices using different technologies or by different organizations, such as a third-party vendor, managing the computing device 600 or associated services based on a proprietary technology.

In one embodiment, the 3D Printing module 334 may be configured to send the virtual 3D model to the printer 336 with instructions for printing the 3D printed model according to the virtual 3D model. The 3D Printing module 334 may therefore trigger the printer 336 to print the virtual 3D model as a physical 3D printed model. In some examples, the 3D Printing module 334 may continuously poll the printer 336 for a print completion signal in order to monitor progress of the 3D printing process. Once the 3D Printing module 334 receives the print completion signal from the printer 336, the 3D Printing module 334 may transmit an indication (e.g., audio, visual, haptic, text-based, symbolic, electromagnetic, voltage/current signal, or any combinations thereof) of such to the display screen 12, AR/VR headsets 20, and/or a remote device.

In a second exemplary embodiment **(****FIG. 8B****),** the system 820 may include the computing device 600, a network device 804, and the printer 336 operating in communication with each other over the network 802. The system 820 may include the 3D Printing module 334 integrated, installed, or operatively associated with the network device 804 (e.g., an access point) configured to establish the network 802 among various other network devices such as standalone computing devices and servers. The network device 804 may be preconfigured or dynamically configured by the processor 620 to provide an interface for assisting the exchange of software instructions and data among the connected devices such as the computing device 600 and the printer 336. In some embodiments, the network device 804 may be preconfigured or dynamically configured to include the 3D Printing module 334 integrated with other devices. For example, the 3D Printing module 334 may be integrated with a remote device such as a server (not shown) or any other computing device connected to the network 802. The server may include a module (not shown), which enables the server for being introduced to the network device 804, thereby enabling the network device 804 to invoke the 3D Printing module 334 as a service. Examples of the network device 804 include, but are not limited to, a DSL modem, a wireless access point, a router, a signal repeater or enhancer, and a gateway having a predetermined computing power and memory capacity sufficient for implementing the 3D Printing module 334. Accordingly, the network device 804 may assist to implement a distributed network architecture, with or without the server, for executing different aspects of the 3D Printing module 334 separately or in tandem through various other connected devices such as the computing device 600 and the printer 336.

In some embodiments, the network device 804 may provide an intermediary medium to the Networking module 310 for communicating with the printer 336, and vice versa. Examples of the intermediary medium may include, but are not limited to, physical connections (e.g., USB, SD cards, ports, cables, etc.), network options (e.g., Wi-Fi^{®}, Bluetooth^{®}, NFC^{®}, virtual port network, etc.), file-based interfaces (e.g., G-code, STL or OBJ files, API/SDK interfaces, etc.), communication protocols, and virtual or real physical optical marker objects such as those mentioned above, or fiducial markers. The Networking module 310 may be configured to send instructions to a remote device such as the printer 336, discussed below in greater detail.

In a third exemplary embodiment **(****FIG. 8C****),** the system 830 may include the computing device 600 in communication with the printer 336 over the network 802. As illustrated, the printer 336 may include the 3D Printing module 334 installed or integrated therewith. The 3D printing module 334 may communicate with the Networking module 310 to control the printer 336 or any remote device. Further, in a fourth embodiment (not shown), the 3D Printing module 334 may operate as an independent, standalone device including its own processor(s), such as that shown in **FIG. 7**, and a transceiver unit (not shown). The 3D Printing module 334 may be implemented as a single dedicated device or that being a combination of multiple dedicated devices. Similar to the computing device 600, the standalone 3D Printing module 334 may be portable or fixed in position. Alternatively, the 3D Printing module 334 may be made mobile or stationary. The 3D Printing module 334 may accordingly communicate directly with the connected devices such as the computing device 600, the network device 804, and the printer 336 over the network 802 using the transceiver unit. Other embodiments may include the 3D Printing module 334, or aspects thereof, being implemented in a decentralized network architecture.

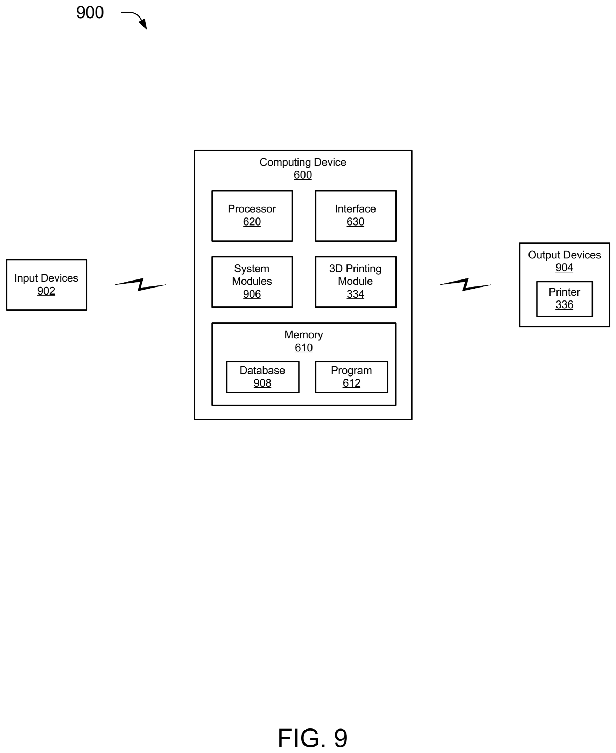

**FIG. 9** illustrates an exemplary system for generating patient-specific implants for anatomical structures of patients, according to an embodiment of the present application. In one embodiment, the system 900 may include the computing device 600 operating in communication with input devices 902 and output devices 904. Examples of the input devices 902 may include, but are not limited to, the haptic device(s) 28, Keyboard & Mouse 14, AR/VR hand controllers 16, 18, AR/VR tracking system 24, 26, the Input data source 332, the network device 804, and medical image scanners such as MRI scanners and CT scanners, as discussed above. Examples of the output devices 904 may include, but are not limited to, the haptic device(s) 28, the AR/VR headset 20, the display screen 12, and the network device 804, as discussed above. As illustrated, the output devices 904 may further include the printer 336 configured to print or generate an output in 2D and/or 3D form or format. Each of the input devices 902 and the output devices 904 may operate in communication with the computing device 600 over the network 802. The computing device 600 may be implemented by way of a single computing device or a combination of multiple devices that may be operatively connected or networked together. The computing device 600 may be implemented in hardware or a suitable combination of hardware and software. In the present embodiment, the computing device 600 may represent a hardware device including the processor 620 executing machine-readable program instructions to perform various functions including those mentioned above. In some embodiments, the computing device 600 may be integrated with the one or more of the input devices 902 and the output devices 904.

The computing device 600 may also include a variety of known, related art, or later developed interfaces, such as the interface 630 including software interfaces (e.g., application programming interfaces, graphical user interfaces, etc.); hardware interfaces (e.g., cable connectors, physical or digital keyboards, card readers, barcode readers, radio frequency identity (RFID) readers, biometric scanners, interactive display screens, transceiver circuits, etc.); or both. The interfaces such as the interface 630 may assist the computing device 600 to communicate with the input devices 902 and the output devices 904. Examples of the interface 630 may include, but are not limited to, the Haptic Interface 330, the AR/VR Interface 324, the AR/VR Graphics User Interface 326, and 2D Graphics User Interface 328, as discussed above.

In one embodiment, the computing device 600 may include one or more system modules 906 and the 3D Printing module 334 operating in communication therewith. Examples of the system modules 906 may include, but are not limited to, the State Change Router 312, the View Controllers 320, the Graphics Rendering module 322, the Undo/Redo module 308, the Networking module 310, the Application State module 304, and the Save/Load module 306, as discussed above.

The computing device 600 may further include the memory 610 for storing, at least one of: (1) files and related data including metadata, e.g., data size, data format, creation date, associated tags or labels, images, documents, messages or conversations, etc.; (2) a log of profiles of network devices and associated communications including instructions, queries, conversations, data, and related metadata; and (3) predefined or dynamically defined, calculated, manipulated, or used mathematical models, 2D and/or 3D software models, equations, or methods for, without limitation, (i) image processing; (ii) visualization in a simulated or virtual environment; (iii) generating 3D printed model; (iv) remote access, retrieval, and/or storage; and so on.

The memory 610 may comprise any computer-readable medium known in the art, related art, or developed later including, for example, a processor or multiple processors operatively connected together, volatile memory (e.g., RAM, cache, etc.), non-volatile memory (e.g., flash, solid state drive, etc.), disk drive, etc., or any combinations thereof. The memory 610 may include one or more databases such as a database 908, which may be sub-divided into further databases for storing electronic files and data. The memory 610 may have one of many database schemas known in the art, related art, or developed later for storing the data, predefined or dynamically defined models, parameters or attributes, and values thereof. For example, the database 908 may have a relational database schema involving a primary key attribute and one or more secondary attributes. In some embodiments, the processor 620 may perform one or more operations including, but not limited to, reading, writing, deleting, indexing, segmenting, labeling, updating, and manipulating the data (e.g., voxel data values, virtual 3D models, etc.), or any combinations thereof, and may communicate the resultant data to one or more of the output devices 904.

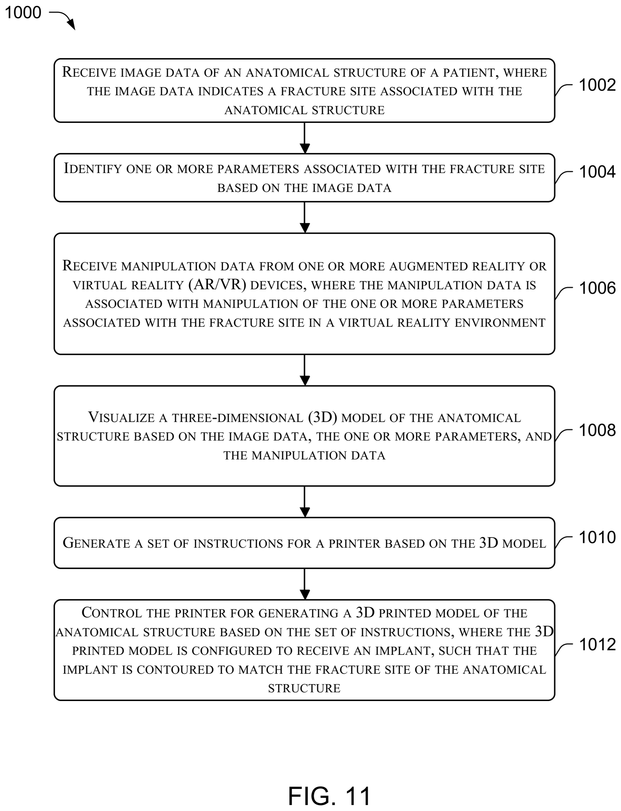

In one embodiment, the memory 610 may include the program instructions 612 to implement various software and hardware components of the computing device 600. For example, the program instructions 612 may be executed by the processor 620 to implement the system modules 906 and the 3D printing module 334. The processor 620 may configure and/or control the system modules 906 and the 3D printing module 334 in communication with the input devices 902 and the output devices 904 via the interface(s) 630 to implement the exemplary method 1000 illustrated in **FIG. 11**. The exemplary method 1000 may be described in the general context of computer executable instructions. Generally, computer executable instructions may include routines, programs, objects, components, data structures, procedures, modules, functions, and the like that perform particular functions or implement particular abstract data types. The computer executable instructions may be stored on a computer readable medium, and installed or embedded in an appropriate device for execution. The order in which the method 1000 is described is not intended to be construed as a limitation, and any number of the described method blocks may be combined or otherwise performed in any order to implement the method 1000, or an alternate method. Additionally, individual blocks may be deleted from the method 1000 without departing from the concepts described herein. Furthermore, the method 1000 may be implemented in any suitable hardware, software, firmware, or combination thereof, that exists in the related art or that is later developed. The method 1000 describes an exemplary implementation of the one or more processors 620. One having ordinary skill in the art would understand that the method 1000 may be modified appropriately for implementation with other configurations and methods or by any of the suitable modules of the one or more processors 620 without departing from the concepts described in the present application.

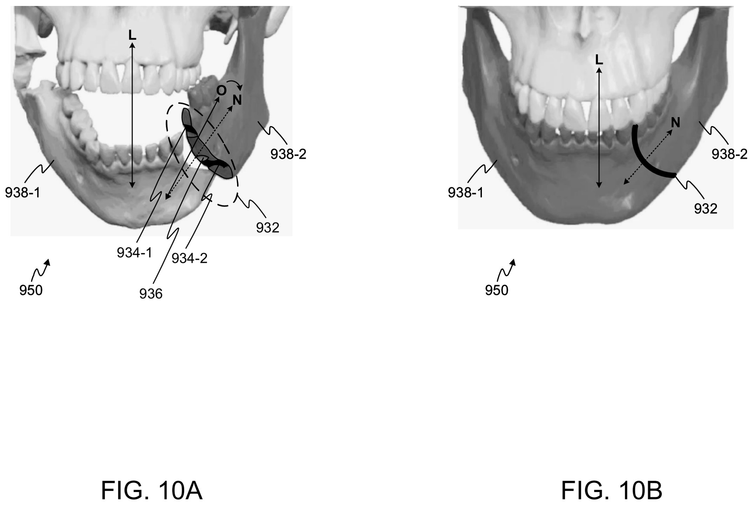

**FIGS. 10A-10B** illustrate an exemplary anatomical structure, such as a bone 950 having a fracture site 932 being manipulated using the computing device 600, according to an embodiment of the present application. Further, **FIG. 11** illustrates a flowchart of a method 1000 for generating patient-specific implants for anatomical structures of patients, according to an embodiment of the present application. To this end, elements of **FIGS. 10A-10B** are explained in conjunction with elements of **FIG. 11** for sake of clarity.

At step 1002, image data of an anatomical structure of a patient is received. In one embodiment, the processor 620 may operate one or more controllers such as the View controllers 320 may execute instructions stored in the memory 610 to query the Application State module 304 for the state of volume and image data. For example, the View controllers 320 may interface with the State Change Router 312 to relay the commands for fetching the image data from the input data source 332. The View controllers 320 may query the Volume and image data system 316 (of the Application State module 304) to receive the image data from the input data source 332. In one example, the View controllers 320 may receive 3D image data from the input data source 332 such as an MRI scanner (not shown). The received 3D image data may be stored in the memory 610. The 3D image data may include one or more volume datasets representing an anatomical structure of the patient. Each of the volume datasets may include voxel data values, which may define the size and dimension of volume objects such as the one or more anatomical structures in the image data. For example, the 3D image data may include a volume dataset representing a jaw bone 950, as illustrated in **FIG. 10A****,** where the volume dataset may include voxel data values defining the size and dimension of the jaw bone 950.

The volume datasets may represent scalar volume datasets, where each voxel in the volume datasets may have to a scalar value. Such scalar voxel values may be collectively referred to as voxel data in the volume datasets. The voxel data may correspond to attributes of voxels in the volume datasets. Examples of the voxel attributes may include, but are not limited to, color, color shade, opacity or transparency, emittance, scalar magnitude, or partial derivatives of gradients. Thus, the received volume datasets may include multiple scalar values, each being associated with a voxel. In one example, each voxel may belong to an anatomical structure, such as the jaw bone 950, of the patient in the received image data. In some examples, the processor 620 may trigger the View controllers 320 to receive 2D image data from one of the input devices 902 such as a CT scanner or any other suitable medical scanners known in the art via the Application State module 304.

In one embodiment, the processor 620 may trigger the View controllers 320 to determine an anatomical structure based on the associated scalar values. For example, the anatomical structure such as the jaw bone 950 may be visualized or displayed in a virtual scene on the display screen 12 and/or the AR headsets 20 using iso-surfaces generated through various techniques implemented by the processor 620 via the Application State module 304. Examples of these techniques may include, but are not limited to, volume meshing algorithm, topological smoothing process, editable volume objects, and surface normals. Each of the iso-surfaces, in one example, may refer to a 3D surface that may represent points of a constant scalar value within a volume dataset. The iso-surfaces are often used in medical imaging and scientific visualization to highlight specific regions of interest, such as the jaw bone 950 or any other anatomical structures in a scanned body represented by the received image data. The processor 620 may extract the iso-surfaces from the scalar volume datasets using a mathematical criterion (e.g., voxels with the same intensity or scalar value).

The processor 620, in one embodiment, may operate the Application State module 304 to determine one or more anatomical structures in the volume datasets. For example, the processor 620 may identify an anatomical structure as the jaw bone 950 based on the corresponding scalar values being above a preset threshold value. The identified anatomical structure may represent a region of interest, e.g., selected for an intended operation. In some examples, the processor 620 may operate, or configure the Application State module 304, to implement either the signed distance function (SDF) or a connected component labeling (CCL) process, as discussed above, to identify and/or select a region of interest in the anatomical structure associated with the one or more volume datasets. The scalar values associated with the anatomical structures may indicate one or more properties of the corresponding anatomical structure. Examples of these properties may include, but are not limited to, an intensity, a density, and a material type (e.g., organic material (e.g., collagen), inorganic material (e.g., hydroxyapatite), water content, etc.) of the anatomical structure.

In one embodiment, the image data may indicate a fracture site associated with an anatomical structure of the patient. For example, the image data may include voxel data representing an anatomical site comprising a fracture site 932, as illustrated in **FIGS. 10A-10B****.** The processor 620, alone or in communication with the View controllers 320, may operate to determine the fracture site 932 **(****FIG. 10A****)** in the anatomical structure such as the jaw bone 950. For instance, the processor 620 may operate, or configure the Application State module 304 in some examples, to determine the fracture site 932 and associated parameters based on the received image data. In one example, the fracture site 932 may correspond to a region of discontinuity in the anatomical structure, i.e., the jaw bone 950. The region of discontinuity (or fracture site 932) may correspond to a break, crack, gap, or any other type of disruption or deviation in the anatomical structure, such as the jaw bone 950, relative to the original or natural structural characteristics of the jaw bone 950 (e.g., lower jaw bone). Examples of the structural characteristics may include, but are not limited to, dimensions, shape, color, opacity or transparency, density, and relative position to another anatomical structure (e.g., upper jaw bone).

In one example, as illustrated in **FIG. 10A****,** the region of discontinuity (or fracture site 932) may include a misalignment or a deformity in an anatomical structure, such as the jaw bone 950. For instance, as shown in **FIG. 10A****,** the jaw bone 950 may include a first portion 938-1 and a second portion 938-2 (hereinafter collectively referred to as 938) offset or separated therefrom across the fracture site 932 with respect to an axis O. The fracture site 932 may correspond to a single fracture (or region of discontinuity) or a set of multiple distinct fractures (e.g., multiple discrete regions of discontinuity) in the anatomical structure, e.g., the jaw bone 950. Some examples may include the fracture site 932 excluding a surrounding or targeted anatomical structure. For instance, a skull fracture involving a cranial bone may exclude brain tissue or meninges. In another instance, a phalangeal fracture in a finger joint may exclude the adjacent tendons or ligaments.

The region of discontinuity (or fracture site) may be determined based on the associated scalar values in the image data. For example, the processor 620 may determine the scalar values associated with the identified anatomical structure (or the region of interest) such as the jaw bone 950. The determined scalar values may be compared with a preset threshold value. The scalar values less than or equal to the preset threshold value for the identified jaw bone 950 may indicate that the fracture site 932 may be located along or in that jaw bone 950.

At step 1004, one or more parameters associated with the fracture site is identified based on the received image data. In one embodiment, the processor 620 may operate the View controllers 320 to execute instructions in the memory 610 for identifying one or more parameters associated with the fracture site 932 (or the region of discontinuity). The fracture site 932 may be represented as scalar values (or absence thereof, or below a set threshold value) in the volume datasets. Each of the scalar values may correspond to one or more parameters of the fracture site 932. The processor 620 may operate the View controllers 320 to determine one or more parameters associated with the fracture site 932 based on the corresponding scalar values. Examples of these parameters may include, but are not limited to, visual properties (e.g., colors, level of transparency, iso-values, transfer functions, special visual effects achieved by shaders, etc.), 2D and 3D measurements of spatial properties (e.g., distance between two points, cumulative length of a polygonal chain, angle between two lines, angle between two planes, circumference of a circle, volumetric size of a space or gap, volumetric size within an iso-surface, etc.), specific region boundaries such as through segmentation including iso-surface thresholds, spatial position and alignment or orientation, voxel attributes (e.g., color, color shade, opacity or transparency, emittance, scalar magnitude, partial derivatives of gradients, etc.), and position of (or proximity to) one or more sites relative to optical markers or fiducial markers.

At step 1006, manipulation data is received from one or more AR/VR devices. In one embodiment, the processor 620 may receive manipulation data from one or more AR/VR devices such as AR/VR hand controllers 16, 18 and AR/VR Headset 20 via one or more interaction interface 630, such as AR/VR interface 324, AR/VR GUI 326, and/or haptic interface 330. The manipulation data may be associated with manipulation of the one or more parameters, such as those mentioned above, associated with the fracture site 932 in the virtual reality environment. For example, the processor 620 may operate, or configure the Application State module 304, to communicate with the graphics rendering module 322 to visualize the fracture site 932 of the jaw bone 950 in a virtual scene on the display screen 12 and/or AR/VR headsets 20. The visualized fracture site 932 (hereinafter also referred to as virtual fracture site 932) may correspond to the actual fracture site in the patient anatomy.

The processor 620 may operate, or configure the Application State module 304, to manipulate the virtual fracture site 932. In one example, the processor 620 may manipulate the virtual fracture site based on a preset alignment of one or more anatomical portions in the virtual environment. For instance, as illustrated in **FIG. 10A****,** the processor 620 may be configured to realign the first portion 938-1 and the second portion 938-2 of the jaw bone 950 across the fracture site 932 based on the preset or known alignment data of the jaw bone 950 stored in the memory 610. The processor 620 may move the second portion 938-2 from the axis O to realign the second portion 938-2 along the axis N with respect to the first portion 938-1. The processor 620 may realign the first and the second portions 938 to have the axis N pass through a geometric center (and/or along a longitudinal axis or oblique axis N) of the jaw bone 950. The processor 620 may associate the realigned anatomical portions, such as the first and/or second portions 938, with the jaw bone 950 having the virtual fracture site 932 in the volume datasets.

In another example, the processor 620 may remove or cull one or more anatomical portions associated with the anatomical structure having the virtual fracture site in the volume datasets. For instance, the processor 620 may be configured to remove or cull an anatomical portion, such as bone fragments 934-1 and 934-2 (collectively referred to as bone fragments 934) and a tissue fragment 936, proximate to the virtual fracture site 932. In one example, the bone fragments 934 and the tissue fragment 936 may extend outward from a cut boundary of the jaw bone 950, where such cut boundary (or edge) may be positioned or oriented towards the fracture site 932, i.e., the region of discontinuity. In some examples, the cut boundary of the jaw bone 950 may include the fracture site 932, or vice versa. The step of removal or culling one or more anatomical portions of the anatomical structure, such as the jaw bone 950, may assist in smoothening the cut boundary to prepare the virtual fracture site 932 for receiving an intended implant (not shown). Upon removal of the bone fragments 934 and the tissue fragment 936, the second portion 938-2 of the jaw bone 950 may be manipulated, e.g., oriented, positioned, and/or carved, to receive the first portion 938-1 of the jaw bone 950, or vice versa. The manipulated jaw bone 950 including the corresponding first and second portions 938 across the virtual fracture site 932 is illustrated in **FIG. 10B****.** In a further example (as shown in **FIG. 10A****),** the processor 620 may modify the cut boundary (or any anatomical portion proximate thereto such as the tissue fragment 936) using a virtual paint to better define or delineate a bone region proximate to the virtual fracture site 932 to provide the manipulated jaw bone 950. In some examples, the processor 620 may be also configured to add at least in-part a preset virtual filler material (e.g., virtual paint, shades, textures, etc.) to the virtual fracture site 932 for manipulation.

In one embodiment, the processor 620 may be configured to operate independently or based on a user input to move or modify one or more anatomical portions of the jaw bone 950 in the virtual environment, as discussed above, to reduce the virtual fracture site 932. For example, the processor 620 may remove the fragments 934, 936 (or fill an associated depression using the preset virtual filler material) followed by moving the second portion 938-2 of the jaw bone 950 closer (or within a preset distance, e.g., less than or equal to 10 millimeter) to the first portion 938-1 of the jaw bone 950 in the virtual environment. Due to the removal or filling of stray or unwanted anatomical portions such as the fragments 934, 936, the first portion 938-1 of the jaw bone 950 may be configured to receive or align substantially proximate to the second portion 938-2 of the jaw bone 950, or vice versa, thereby reducing the virtual fracture site 932 in the jaw bone 950, as illustrated in **FIG. 10B****.** In a further example, as illustrated in **FIG. 10B****,** the processor 620 may move the jaw bone 950, such as a lower jaw bone, along a vertical axis L (or a central axis of a jaw bone or the skull) to receive or come in contact with the upper jaw bone, or vice versa.

In another example, a user may interact with the virtual fracture site 932 via the AR/VR hand controllers 16, 18 to manually remove one or more anatomical portions (e.g., fragments 934, 936), realign the one or more anatomical portions (e.g., first and second portions 938 of the jaw bone 950), modify the cut boundary or any other anatomical portion in or around the fracture site 932, and/or at least in-part fill the virtual fracture site 932 using a virtual paint in the virtual environment. Such manipulation of the virtual fracture site 932 may assist in (i) defining a clear boundary and/or an edge(s) of the fracture site 932 (or the connected anatomical structure) in the virtual scene, and (ii) providing an estimate of open space available in and/or around the virtual fracture site to prepare for installing or mounting (a) an intended implant and/or (b) a preset fixture configured to receive the implant thereon.

The manipulation of the fracture site 932 and/or proximate anatomical portions of the jaw bone 950 may adjust or modify the corresponding scalar values associated with the one or more parameters of the fracture site 932. The modified scalar values may constitute the manipulation data associated with the parameters, which may be manipulated to reduce the intensity of the fracture site 932. The manipulation data may be stored in the memory 610 for future access and/or retrieval.

At step 1008, a three-dimensional (3D) model of the anatomical structure may be visualized. In one embodiment, the processor 620 may operate, or configure the Application State module 304, to communicate with the graphics rendering module 322 to visualize the manipulated fracture site 932 in a virtual scene on the display screen 12, a GUI such as the AR/VR GUI 326, and/or AR/VR headsets 20. The graphics rendering module 322 may operate in communication with the processor 620 to perform one or more operations. For example, the graphics rendering module 322 may determine and generate a volumetric dataset of the anatomical structure, such as the jaw bone 950, including the manipulated fracture site based on the one or more parameters of the fracture site 932 and the manipulation data. The graphics rendering module 322 may use the determined volumetric dataset to extract the iso-surfaces to define boundaries of the volume objects such as the anatomical structure including the manipulated virtual fracture site 932. The graphics rendering module 322 may also perform the process of volume meshing on the determined volumetric dataset to generate the surface geometries, which may be smoothened and refined by the graphics rendering module 322 using the noise reduction algorithm to generate the virtual 3D model of the manipulated anatomical structure, such as the jaw bone 950 **(****FIG. 10B****),** including the manipulated virtual fracture site 932. In some examples, the generated virtual 3D model may also exclude or hide the manipulated virtual fracture site. Other examples may include generated virtual 3D model having a 3D representation of the patient including the manipulated anatomical structure, such as the jaw bone 950 **(****FIG. 10B****),** with or without the manipulated virtual fracture site 932.

The processor 620, via the graphics rendering module 322, may render the virtual 3D model of the manipulated fracture site 932 **(****FIG. 10B****),** or the corresponding manipulated anatomical structure such as the jaw bone 950 **(****FIG. 10B****),** on the display screen 12 or the AR/VR headsets 20. The generated virtual 3D model may be visualized in a 3D virtual space using any of the various graphics rendering mechanisms known in the art, related art, or developed later, including those mentioned above. In further examples, the processor 620, via the Save/Load module 306, may save or store the generated virtual 3D model of the manipulated fracture site 932 **(****FIG. 10B****),** or the corresponding manipulated anatomical structure such as the jaw bone 950 **(****FIG. 10B****),** in the memory 610 or a remote device for future access and/or retrieval.

At step 1010, a set of instructions for a printer is generated. In one embodiment, the processor 620 may operate, or configure the Networking module 310, to communicate with the output devices 904 and remote devices, such as those mentioned above. For example, the processor 620 may configure the Networking module 310 or the 3D Printing module 334, to generate a set of instructions for a printer 336 based upon generation of the virtual 3D model, discussed above. In some examples, the Networking module 310 may include the 3D Printing module 334, or vice versa. Other examples may include the Networking module 310 being configured to trigger the 3D Printing module 334 to generate the set of instructions for the printer 336 based on the generated virtual 3D model. In further examples, the set of instructions may be accessed from the memory 610 or generated by the 3D Printing module 334 in communication with a remote computing device, e.g., without limitation, the network device 804.

At step 1012, the printer is controlled for generating a 3D printed model. In one embodiment, the 3D printing module 334 may be configured to control the printer, such as the printer 336, based on the generated set of instructions. For example, the 3D printing module 334 may configure the printer 336 to (i) fetch the virtual 3D model of the manipulated fracture site 932 (as shown in **FIG. 10B****),** or the corresponding manipulated anatomical structure such as the jaw bone 950 (as shown in **FIG. 10B****),** or that of the underlying patient, from the storage medium such as the memory 610 or a remote device, and (ii) generate a 3D printed model of the manipulated fracture site 932 (as shown in **FIG. 10B****),** or the corresponding manipulated anatomical structure such as the jaw bone 950 (as shown in **FIG. 10B****),** or that of the underlying patient, based on the related virtual 3D model. In some examples, the 3D printing module 334 may send the virtual 3D model to the printer 336 directly or via a remote device, such as those mentioned above. The 3D printed model may include or represent a physical 3D model of the manipulated fracture site 932 (as shown in **FIG. 10B****),** or the corresponding anatomical structure such as the jaw bone 950 (as shown in **FIG. 10B****),** or that of the patient, included therewith.

In one embodiment, the 3D printed model may be configured to receive a predetermined implant (not shown) or any other suitable restorative device known in the art, related art, or developed later including prosthetics. In some examples, the 3D printed model may be configured to mount the preset fixture thereto. The implant may be mounted on the 3D printed model either directly or via the preset fixture.

The implant may be adapted to mount on the 3D printed model in a predetermined manner that further reduces the intensity of the region of discontinuity (or the fracture site 932) in the corresponding anatomical structure, such as the jaw bone 950. The predetermined manner may relate to (i) implant attributes, (ii) implant configuration, (iii) type of implant mounting mechanism, (iv) a position on the anatomical structure relative to the fracture site (or vice versa), (v) a proximity to an adjacent (or remote) anatomical structure, (vi) a proximity to an adjacent (or remote) fracture site, and/or (vii) a distance to or from an edge or end of an anatomical structure or a fracture site. Examples of the implant attributes may include, but are not limited to, size, shape, dimensions (e.g., length breadth, height or thickness, etc.), material type or composition, surface properties (e.g., porosity, hydrophobic or hydrophilic coatings, etc.), strength, a number and type of moveable and stationary parts, a number and type of removable parts, a probability or a measure of toxicity over time, average lifespan or durability, and flexibility or rigidity.

Similarly, examples of the implant configuration may include, but are not limited to, orientation (e.g., specific angle or alignment with respect to the facture site 932 or anatomical structure such as the jaw bone 950), single component or multi-component assembly, and static (or fixed) or dynamic (or movable) functionality. Examples of the type of implant mounting mechanism may include, but are not limited to, screw-fit, press-fit, cemented mounting, anchored or pegged mounting, sutured or tethered mounting, snap-fit, magnetic mounting, adhesive mounting, and integrated or grow-in mounting. In some examples, the implant includes one or more portions that may be bent or bendable to substantially match, align with, or cover the fracture site, such as the fracture site 932, of the anatomical structure such as the jaw bone 950 based on the 3D printed model. Examples of the implant may include, but are not limited to, a plate, a screw, and a rod. Further, in some examples, the implant may be contoured to substantially match, align with, or cover the actual fracture site of the anatomical structure in the patient anatomy. The contoured implant, which may be matched or aligned with the fracture site of the anatomical structure, may be positioned or configurable to be positioned on the actual fracture site of the patient.

In one embodiment, the implant may be mounted on or proximate the fracture site or the corresponding anatomical structure, which may belong to the 3D printed model or the patient anatomy, in the predetermined manner, such as that discussed above, to reduce the intensity of the region of discontinuity (or the fracture site) in the corresponding anatomical structure. This predetermined manner may represent or provide a patient-specific arrangement for positioning the implant on to or with the fracture site in the 3D printed model. This patient-specific arrangement for the implant may be implemented or referred to by a user to position or reposition the implant on the corresponding actual fracture site of the patient anatomy for a better fit.

In some embodiments, the 3D printed model mounted with the implant (hereinafter also referred to as mounted model) may be scanned using medical scanners for the processor 620, e.g., via the Application State module 304, to generate a new virtual 3D model of the mounted model, in a manner as discussed above. The processor 620 may store the new virtual 3D model in the memory 610 or a remote storage device or medium for future access and/or retrieval. In some examples, the new virtual 3D model may be used by the processor 620 to enable visualization of the mounted model, and/or interaction therewith, in the virtual tool.

Thus, the method 1000 enables to reduce surgery time, ensures higher success rate and reduces patient discomfort. The patient need not be put under anesthesia for longer time. Based on reduction in the intensity of the fracture site in the virtual environment, the doctors may be continuously guided to perform the same on patient to reduce risks. As the implants are first arranged on the 3D printed model, the implants may be adapted to be patient specific and can be quickly arranged on the patient's fracture site during surgery.

The above description does not provide specific details of the manufacture or design of the various components. Those of skill in the art are familiar with such details, and unless departures from those techniques are set out, techniques, known, related art or later developed designs and materials should be employed. Those in the art are capable of choosing suitable manufacturing and design details.

Note that throughout the following discussion, numerous references may be made regarding servers, services, engines, modules, interfaces, portals, platforms, or other systems formed from or using computing devices. It should be appreciated that the use of such terms is deemed to represent one or more computing devices having at least one processor, such as the processors 620, configured to or programmed to execute software instructions stored on a computer readable tangible, non-transitory medium or also referred to as a processor-readable medium. For example, a server can include one or more computers operating as a web server, database server, or other type of computer server in a manner to fulfill described roles, responsibilities, or functions. Within the context of this document, the disclosed devices or systems are also deemed to comprise computing devices having a processor and a non-transitory memory storing instructions executable by the processor that cause the device to control, manage, or otherwise manipulate the features of the devices or systems.

Some portions of the detailed description herein are presented in terms of algorithms and symbolic representations of operations on data bits performed by conventional computer components, including a central processing unit (CPU), memory storage devices for the CPU, and connected display devices. These algorithmic descriptions and representations are the means used by those skilled in the data processing arts to most effectively convey the substance of their work to others skilled in the art. An algorithm is generally perceived as a self-consistent sequence of steps leading to a desired result. The steps are those requiring physical manipulations of physical quantities. Usually, though not necessarily, these quantities take the form of electrical or magnetic signals capable of being stored, transferred, combined, compared, and otherwise manipulated. It has proven convenient at times, principally for reasons of common usage, to refer to these signals as bits, values, elements, symbols, characters, terms, numbers, or the like.

It should be understood, however, that all of these and similar terms are to be associated with the appropriate physical quantities and are merely convenient labels applied to these quantities. Unless specifically stated otherwise, as apparent from the discussion herein, it is appreciated that throughout the description, discussions utilizing terms such as "receiving" or "providing" or "determining" or "identifying" "or visualizing" or "comparing" or "storing" or "selecting" or "generating" or "changing" or "modifying" or the like, refer to the action and processes of a computer system, or similar electronic computing device, that manipulates and transforms data represented as physical (electronic) quantities within the computer system's registers and memories into other data similarly represented as physical quantities within the computer system memories or registers or other such information storage, transmission or display devices.

The exemplary embodiments also relate to an apparatus for performing the operations discussed herein. This apparatus may be specially constructed for the required purposes, or it may comprise a general-purpose computer selectively activated or reconfigured by a computer program stored in the computer. Such a computer program may be stored in a computer readable storage medium, such as, but is not limited to, any type of disk including floppy disks, optical disks, CD-ROMs, and magnetic-optical disks, read-only memories (ROMs), random access memories (RAMs), EPROMs, EEPROMs, magnetic or optical cards, or any type of media suitable for storing electronic instructions, and each coupled to a computer system bus.

The algorithms and displays presented herein are not inherently related to any particular computer or other apparatus. Various general-purpose systems may be used with programs in accordance with the teachings herein, or it may prove convenient to construct more specialized apparatus to perform the methods described herein. The structure for a variety of these systems is apparent from the description above. In addition, the exemplary embodiment is not described with reference to any particular programming language. It will be appreciated that a variety of programming languages may be used to implement the teachings of the exemplary embodiment as described herein.

The methods illustrated throughout the specification, may be implemented in a computer program product that may be executed on a computer. The computer program product may comprise a non-transitory computer-readable recording medium on which a control program is recorded, such as a disk, hard drive, or the like. Common forms of non-transitory computer-readable media include, for example, floppy disks, flexible disks, hard disks, magnetic tape, or any other magnetic storage medium, CD-ROM, DVD, or any other optical medium, a RAM, a PROM, an EPROM, a FLASH-EPROM, or other memory chip or cartridge, or any other tangible medium from which a computer can read and use.

Alternatively, the methods may be implemented in transitory media, such as a transmittable carrier wave in which the control program is embodied as a data signal using transmission media, such as acoustic or light waves, such as those generated during radio wave and infrared data communications, and the like.

The terminology used herein is for the purpose of describing particular embodiments only and is not intended to be limiting of the disclosure. It will be appreciated that several of the above-disclosed and other features and functions, or alternatives thereof, may be combined into other systems or applications. Various presently unforeseen or unanticipated alternatives, modifications, variations, or improvements therein may subsequently be made by those skilled in the art without departing from the scope of the present disclosure as encompassed by the following claims.

The claims, as originally presented and as they may be amended, encompass variations, alternatives, modifications, improvements, equivalents, and substantial equivalents of the embodiments and teachings disclosed herein, including those that are presently unforeseen or unappreciated, and that, for example, may arise from applicants/patentees and others.

Also disclosed herein are the following numbered clauses:
1. A system comprising:
   a memory configured to store computer-executable instructions;
   a plurality of controllers configured to execute the instructions to:
      receive image data of an anatomical structure of a patient, wherein the image data indicates a fracture site associated with the anatomical structure; and
      identify one or more parameters associated with the fracture site based on the image data;
   an interaction interface configured to receive manipulation data from one or more augmented reality or virtual reality (AR/VR) devices, wherein the manipulation data is associated with manipulation of the one or more parameters associated with the fracture site in a virtual reality environment, and wherein the one or more parameters are manipulated to reduce an intensity of the fracture site;
   a graphics rendering module configured to:
      receive the image data, the one or more parameters, and the manipulation data,
      visualize a three-dimensional (3D) model of the anatomical structure based on the image data, the one or more parameters, and the manipulation data, wherein the 3D model of the patient is visualized in a 3D space using a plurality of graphics rendering mechanisms, and
      display the visualization of the 3D model on at least one of: a display device, or a GUI; and
   a 3D printing module configured to:
      generate a set of instructions for a printer based on the 3D model, and
      control the printer for generating a 3D printed model of the anatomical structure based on the set of instructions, wherein the 3D printed model is configured to receive an implant, such that the implant is contoured to match the fracture site of the anatomical structure.
2. The system of clause 1, wherein the manipulation data for the one or more parameters associated with the fracture site is received based on at least one of: a user input, or a preset realignment of one or more anatomical portions associated with the anatomical structure having the fracture site.
3. The system of clause 1 or 2, wherein the manipulation data for the one or more parameters is associated with a removal of one or more anatomical portions associated with the anatomical structure having the fracture site.
4. The system of any preceding clause, wherein the manipulation data for the one or more parameters is associated with an insertion of a filler material in the fracture site.
5. The system of any preceding clause, wherein the contoured implant matched with the fracture site of the anatomical structure is configurable to be positioned on the fracture site of the patient.
6. The system of any preceding clause, wherein the implant is one of: a plate, a screw, or a rod.
7. The system of any preceding clause, wherein the implant is bendable to match the fracture site of the anatomical structure.
8. A method comprising:
   receiving image data of an anatomical structure of a patient, wherein the image data indicates a fracture site associated with the anatomical structure;
   identifying one or more parameters associated with the fracture site based on the image data;
   receiving manipulation data from one or more augmented reality or virtual reality (AR/VR) devices, wherein the manipulation data is associated with manipulation of the one or more parameters associated with the fracture site in a virtual reality environment, and wherein the one or more parameters are manipulated to reduce an intensity of the fracture site;
   visualizing a three-dimensional (3D) model of the anatomical structure based on the image data, the one or more parameters, and the manipulation data, wherein the 3D model of the patient is visualized in a 3D space using a plurality of graphics rendering mechanisms; and
   generating a set of instructions for a printer based on the 3D model, and
   controlling the printer for generating a 3D printed model of the anatomical structure based on the set of instructions, wherein the 3D printed model is configured to receive an implant, such that the implant is contoured to match the fracture site of the anatomical structure.
9. The method of clause 8, wherein the manipulation data for the one or more parameters associated with the fracture site is received based on at least one of: a user input, or a preset realignment of one or more anatomical portions associated with the anatomical structure having the fracture site.
10. The method of clause 8 or 9, wherein the manipulation data for the one or more parameters is associated with a removal of one or more anatomical portions associated with the anatomical structure having the fracture site.
11. The method of any of clauses 8 to 10, wherein the manipulation data for the one or more parameters is associated with an insertion of a filler material in the fracture site.
12. The method of any of clauses 8 to 11, wherein the contoured implant matched with the fracture site of the anatomical structure is configurable to be positioned on the fracture site of the patient.
13. The method of any of clauses 8 to 12, further comprising:
   bending at least one of one or more portions of the implant to match the fracture site of the anatomical structure based on the 3D printed model; and
   positioning the implant matched with the fracture site of the anatomical structure on the fracture site of the patient.
14. The method of any of clauses 8 to 13, wherein the implant is one of: a plate, a screw, or a rod.

## Claims

1. A system comprising:
a memory configured to store computer-executable instructions;
a plurality of controllers configured to execute the instructions to:
receive image data of an anatomical structure of a patient, wherein the image data indicates a fracture site associated with the anatomical structure; and
identify one or more parameters associated with the fracture site based on the image data;
an interaction interface configured to receive manipulation data from one or more augmented reality or virtual reality (AR/VR) devices, wherein the manipulation data is associated with manipulation of the one or more parameters associated with the fracture site in a virtual reality environment, and wherein the one or more parameters are manipulated to reduce an intensity of the fracture site;
a graphics rendering module configured to:
receive the image data, the one or more parameters, and the manipulation data,
visualize a three-dimensional (3D) model of the anatomical structure based on the image data, the one or more parameters, and the manipulation data, wherein the 3D model of the patient is visualized in a 3D space using a plurality of graphics rendering mechanisms, and
display the visualization of the 3D model on at least one of: a display device, or a GUI; and
a 3D printing module configured to:
generate a set of instructions for a printer based on the 3D model, and
control the printer for generating a 3D printed model of the anatomical structure based on the set of instructions, wherein the 3D printed model is configured to receive an implant, such that the implant is contoured to match the fracture site of the anatomical structure.

2. The system of claim 1, wherein the manipulation data for the one or more parameters associated with the fracture site is received based on at least one of: a user input, or a preset realignment of one or more anatomical portions associated with the anatomical structure having the fracture site.

3. The system of claim 1 or 2, wherein the manipulation data for the one or more parameters is associated with a removal of one or more anatomical portions associated with the anatomical structure having the fracture site.

4. The system of any preceding claim, wherein the manipulation data for the one or more parameters is associated with an insertion of a filler material in the fracture site.

5. The system of any preceding claim, wherein the contoured implant matched with the fracture site of the anatomical structure is configurable to be positioned on the fracture site of the patient.

6. The system of any preceding claim, wherein the implant is one of: a plate, a screw, or a rod.

7. The system of any preceding claim, wherein the implant is bendable to match the fracture site of the anatomical structure.

8. A method comprising:
receiving image data of an anatomical structure of a patient, wherein the image data indicates a fracture site associated with the anatomical structure;
identifying one or more parameters associated with the fracture site based on the image data;
receiving manipulation data from one or more augmented reality or virtual reality (AR/VR) devices, wherein the manipulation data is associated with manipulation of the one or more parameters associated with the fracture site in a virtual reality environment, and wherein the one or more parameters are manipulated to reduce an intensity of the fracture site;
visualizing a three-dimensional (3D) model of the anatomical structure based on the image data, the one or more parameters, and the manipulation data, wherein the 3D model of the patient is visualized in a 3D space using a plurality of graphics rendering mechanisms; and
generating a set of instructions for a printer based on the 3D model, and
controlling the printer for generating a 3D printed model of the anatomical structure based on the set of instructions, wherein the 3D printed model is configured to receive an implant, such that the implant is contoured to match the fracture site of the anatomical structure.

9. The method of claim 8, wherein the manipulation data for the one or more parameters associated with the fracture site is received based on at least one of: a user input, or a preset realignment of one or more anatomical portions associated with the anatomical structure having the fracture site.

10. The method of claim 8 or 9, wherein the manipulation data for the one or more parameters is associated with a removal of one or more anatomical portions associated with the anatomical structure having the fracture site.

11. The method of any of claims 8 to 10, wherein the manipulation data for the one or more parameters is associated with an insertion of a filler material in the fracture site.

12. The method of any of claims 8 to 11, wherein the contoured implant matched with the fracture site of the anatomical structure is configurable to be positioned on the fracture site of the patient.

13. The method of any of claims 8 to 12, further comprising:
bending at least one of one or more portions of the implant to match the fracture site of the anatomical structure based on the 3D printed model; and
positioning the implant matched with the fracture site of the anatomical structure on the fracture site of the patient.

14. The method of any of claims 8 to 13, wherein the implant is one of: a plate, a screw, or a rod.
